(19) Europäisches Patentamt
European Patent Office
Office européen des brevets

(11) Publication number: **0 288 888 B1**

(12) **EUROPEAN PATENT SPECIFICATION**

(45) Date of publication of patent specification: **08.12.93**

(51) Int. Cl.[5]: **C07H 13/06**, C07H 11/00, C07H 15/04

(21) Application number: **88106313.5**

(22) Date of filing: **20.04.88**

(54) **Novel glucopyranose derivatives.**

(30) Priority: **01.05.87 JP 106298/87**

(43) Date of publication of application:
**02.11.88 Bulletin 88/44**

(45) Publication of the grant of the patent:
**08.12.93 Bulletin 93/49**

(84) Designated Contracting States:
**AT BE CH DE ES FR GB GR IT LI LU NL SE**

(56) References cited:
**EP-A- 0 226 381**
**WO-A-84/04526**
**US-A- 4 374 831**

**AGRICULTURAL AND BIOLOGICAL CHEMISTRY, vol. 48, no. 1, January 1984, The Agricultural Chem. Soc. of Japan; M. KISO et al., pp. 251-252&NUM;**

**CHEMICAL & PHARMACEUTICAL BULLETIN, vol. 33, no. 10, October 1985, Pharm. Soc. of Japan; T. SHIMIZU et al., pp. 4621-4624&NUM;**

(73) Proprietor: **ONO PHARMACEUTICAL CO., LTD.**
**No. 14, Doshomachi 2-chome**
**Higashi-ku**
**Osaka-shi Osaka(JP)**

(72) Inventor: **Toda, Masaaki**
**3-16-12, Todai-ji**
**Shimamoto-cho**
**Mishima-gun Osaka(JP)**
Inventor: **Sasaki, Yutaro**
**88-504, Okayamate-cho 11**
**Hirakata-shi Osaka(JP)**
Inventor: **Shimoji, Katsuichi**
**1-3, RUNE-Shimogamo-Higashi 305**
**Takano-tadeharacho 1, Sakyo-ku**
**Kyoto-shi(JP)**

(74) Representative: **Henkel, Feiler, Hänzel & Partner**
**Möhlstrasse 37**
**D-81675 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 288 888 B1

## Description

### Summary

This invention relates to novel glucopyranose derivatives.

More particularly, this invention relates to novel glucopyranose derivatives which possess activities like Lipid A (described hereafter),
processes for the preparation of them, and
pharmaceutical agent containing them as active ingredient.

### Background

Gram-negative germ (e.g. cholera germ, salmonella germs, colon bacillus) have compounds so called as lipopolysaccharide (abbreviated as LPS) on the outer cell membrane, and it was thought that the compounds induced endotoxin shock.

It was known that LPS has various bioactivities included fetal toxicity, that is why it is so named endotoxin. For example, LPS has pyrogenic action, hemorrhage action, induces encephalomyelitis, arthritis, and has a blastogenic action (macrophage activating action, B cell mitogenic activity, producing action of non-specific antibody, enhancing activity of cellular immunity etc.) and anti-tumor action (INF (interferon) inducing action, TNF (tumor necrosis factor) including action etc.).

Especially, LPS is effective as non-specific immunity agent, and has an action inducing hemorrhage necrosis of tumor cell specifically by its TNF inducing activity, and therefore it can be useful as an anti-tumor agent.

And LPS is also useful for its inducing activity of IL-1 (interleukin-1) or interferon, not only their enhancing activity of cellular immunity but also stimulating of NK (natural killer) activity.

On the other hand, LPS is constituted from three kinds of materials i.e. acidic protein, macromolecular polysaccharides and phospholipid, and phospholipid have been found as its active site by Westphal, Lüdertz et al.

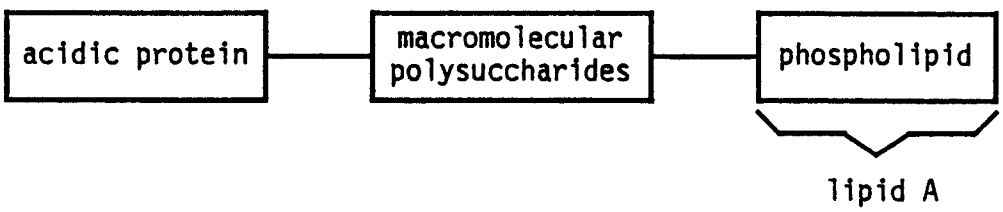


The phospholipid shown above is called lipid A, and it was known that the compound alone possesses various activities like LPS.

The absolute structure of lipid A has been unknown for long years, but recent studies cleared that its structure is a disaccharideamine combined with fatty acids and phosphoric acids as the following [See Nippon Saikin-gaku Zasshi 40(1), 57 (1985) and Proc. Natl. Acad. Sci. U.S.A. 80, 4624 (1983)].

Lipid A of E. Coli

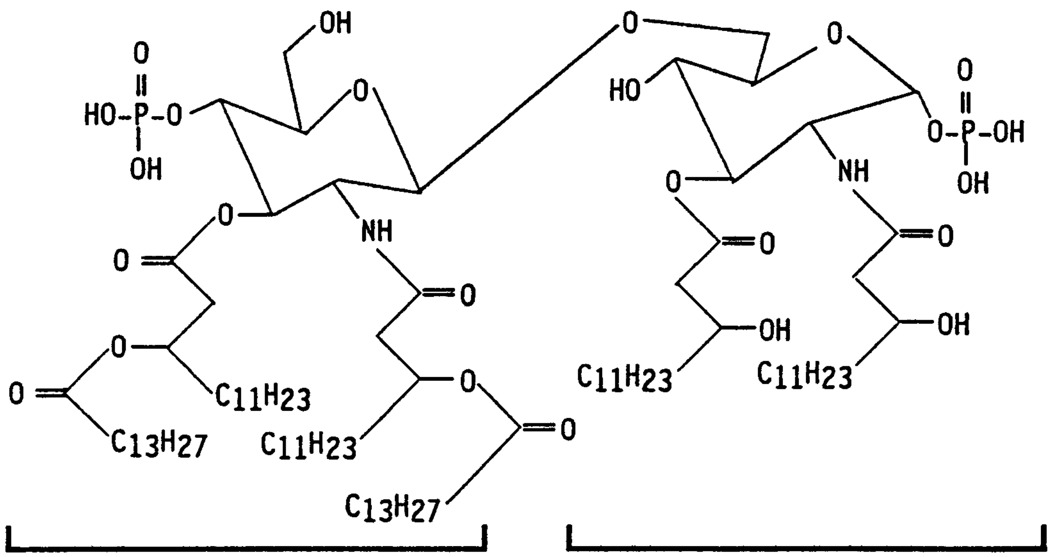

non-reducing subunit reducing subunit

Results of the recent studies revealed that each subunit above possesses activities like Lipid A. And especially, a reducing subunit was isolated as a biosynthesis precursor and was named lipid X [See Biol. Chem. 256, 10690 (1981) and Proc. Natl. Acad. Sci. 80, 4624 (1983)].

And a compound combined with hexadecanoyl group on the hydroxy group of β-hydroxytetradecanoyl group on the 2nd position of lipid X as ester was named lipid Y.

About the above lipid A, lipid X and lipid Y, Wisconsin Alumni Research Foundation filed patent applications WO-8404526 and EP-143840.

Non-reducing subunits were not prepared from natural sources, and some of them were synthesized chemically, i.e. compounds of general formula:

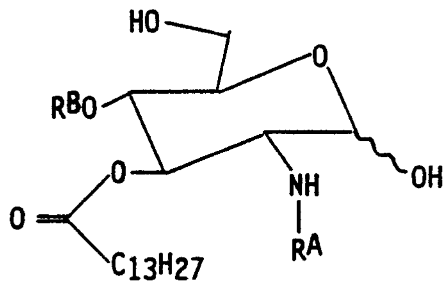

Compound 1: $R^A$ = 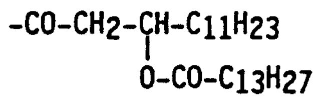

$R^B$ = -H

Compound 2: $R^A$ = $-CO-C_{13}H_{27}$

$R^B$ = $-PO(OH)_2$

Compound 3: $R^A$ = 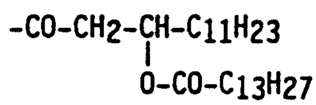

$R^B$ = $-PO(OH)_2$

[See Agric. Biol. Chem., 48(1), 251 (1984) and FEBS LETT., 167, 226 (1984)].

Patent applications related to the above compounds were published [See Japanese Patent Publication No. 61-126093 and 61-126094].

Further, compounds in which the hydroxy group on the 1st position is replaced with a hydrogen atom were published, i.e. compounds of general formula:

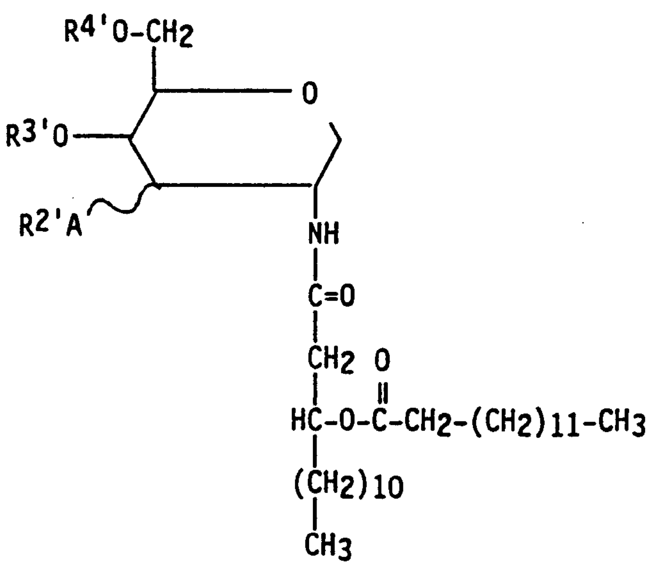

[wherein A represents O or NH, $R^{2'}$ represents $C_{14}$ or $C_{14}$-O-$C_{14}$, $R^{3'}$ and $R^{4'}$ represent a hydrogen atom or P.

$C_{14}$ represents tetradecanoyl group, $C_{14}$-O-$C_{14}$ represents (3-tetradecanoyl)-tetradecanoyl group, and P represents phosphoryl group, respectively. Stereo configuration of $AR^{2'}$ on the 3rd position is $\alpha$-configuration or $\beta$-configuration.]

(See Japanese Patent Publication No. 61-172867).

Disclosure of the Invention

We, the present inventors, succeeded in synthesizing novel compounds wherein the following chemical modifications into the non-reducing subunit have been made :

(1) conversion of the phosphoric acid residue at the 4-position into a sulfuric acid residue,

(2) conversion of the hydroxymethyl group at the 5-position into a hydrogen atom or a sulfoxymethyl group ($-CH_2OSO_3H$), and/or

(3) introduction of an aryl group into the middle or terminal of the acyl chain at the 2- or 3-position,

and we confirmed that they possess pharmacological activities like Lipid-A, and then achieved the present invention.

Therefore, the present invention is related to the novel glucopyranose derivatives of general formula:

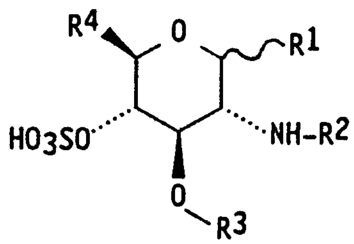

(I)

wherein $R^1$ represents a hydrogen atom, a hydroxy group or an alkoxy group of from 1 to 4 carbon atom(s), $R^2$ represents the group represented by A, B, D or E, wherein

A represents a general formula:

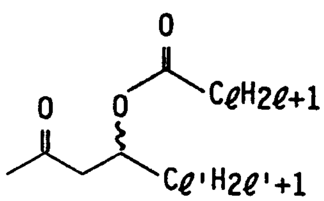

,

B represents a general formula:

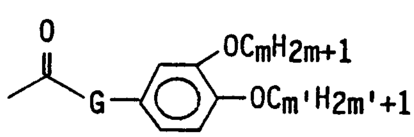

,

D represents a general formula:

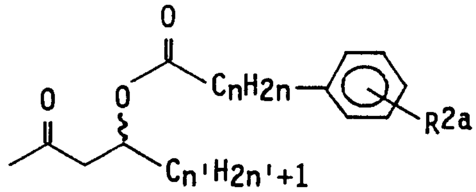

and
E represents a general formula:

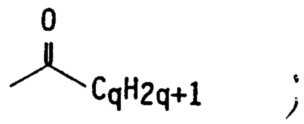

in each formula, ℓ and q each represent an integer of 11~15, m and m′ each represents an integer of 6~12, n represents an integer of 6~10, ℓ ′ and n′ each represents an integer of 9~13,
G represents a single bond or an alkylene group of from 1 to 4 carbon atom(s),
$R^{2a}$ represents a hydrogen atom, an alkyl group or alkoxy group of from 1 to 7 carbon atom(s) or a halogen atom.)]
$R^3$ represents the group represented by L, M or Q , wherein
L represents a general formula:

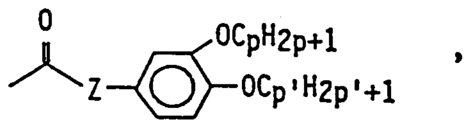

M represents a general formula:

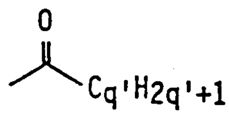

and
Q represents a general formula:

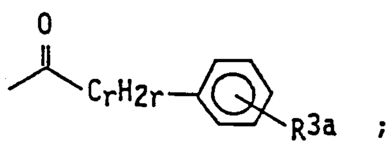

in each formula, Z represents a single bond or an alkylene group of from 1 to 4 carbon atom(s), p and p' each represents an integer of 6~12,
q' represents an integer of 11~15, r represents an integer of 6~10,
$R^{3a}$ represents a hydrogen atom, an alkyl group or alkoxy group of from 1 to 7 carbon atom(s) or a halogen atom.
$R^4$ represents a hydrogen atom, a hydroxymethyl group or a sulfoxymethyl ($-CH_2OSO_3H$) group;
with the proviso that, when $R^4$ represents a hydroxymethyl group
(i) (A,M), (A,Q), (D,M), (D,Q), (E,M) and (E,Q) as the combination of ($R^2$, $R^3$) are excluded
(ii) m and m' in the formula (B) of $R^2$ represent an integer of 8 to 12,
(iii) p and p' in the formula (L) of $R^3$ represent an integer of 8 to 12,
and when $R^4$ represents a sulfoxymethyl group, (A,M) and (E,M) as the combination of ($R^2$, $R^3$) are excluded,
or non-toxic salts thereof, processes for the preparation of them and immunity enhancing agents and/or anti-tumor agents containing them as active ingredients.

In the general formula (I), examples of the alkoxy group of from 1 to 4 carbon atom(s) as $R^1$ are methoxy, ethoxy, propoxy and butoxy groups and isomeric groups thereof, and preferable groups as $R^1$ are a hydrogen atom, a hydroxy group and a methoxy group.

In the groups represented by $R^2$ and $R^3$ in the general formula (I):

(i) the alkyl groups represented by $-C_{\ell}H_{2\ell+1}$, $-C_qH_{2q+1}$ and $-C_{q'}H_{2q'+1}$ are undecyl, dodecyl, tridecyl, tetradecyl and pentadecyl group, of which number of the carbon is from 11 to 15, and isomeric groups thereof, and more preferable group is tridecyl group;

(ii) the alkyl groups represented by $-C_mH_{2m+1}$, $-C_{m'}H_{2m'+1}$, $-C_pH_{2p+1}$ and $-C_{p'}H_{2p'+1}$ are hexyl, heptyl, octyl, nonyl, decyl, undecyl and dodecyl group, of which the number of the carbon atoms is from 6 to 12, and isomeric groups thereof, and more preferable group is decyl group;

(iii) the alkylene groups represented by $-C_nH_{2n}$ and $-C_rH_{2r}$ are hexamethylene, heptamethylene, octamethylene, nonamethylene and decamethylene group, of which the number of the carbon atoms is from 6 to 10, and isomeric groups thereof, and more preferable group is octamethylene group;

(iv) the alkylene group of from 1 to 4 carbon atom(s) as G and Z, are methylene, ethylene, propylene and butylene group and isomeric groups thereof, and a more preferable group is a single bond;

(v) the alkyl groups of from 1 to 7 carbon atom(s) as $R^{2a}$ and $R^{3a}$, are methyl, ethyl, propyl, butyl, pentyl, hexyl and heptyl group, and isomeric groups thereof, and the alkoxy groups of from 1~7 carbon atom(s) are methoxy, ethoxy, propoxy, butoxy, pentyloxy, hexyloxy and heptyloxy group and isomeric groups thereof, and the halogen atoms are fluorine, chlorine, bromine and iodine atoms.

Preferable groups as $R^{2a}$ and $R^{3a}$ are a hydrogen atom.

In the general formula (I), the alkyl groups represented by $-C_{\ell'}H_{2\ell'+1}$ and $-C_{n'}H_{2n'+1}$ in the groups represented by $R^2$, are nonyl, decyl, undecyl, dodecyl and tridecyl, of which the number of carbon atoms is from 9 to 13, and isomeric groups thereof, a more preferable group is undecyl group.

More preferable groups as $R^2$ and $R^3$ each are the group represented by B and L.

In the general formula (I), all of the groups as $R^4$ are preferable.

The alkyl, alkylene and alkoxy groups foregoing described mean straight ones and branched ones, and preferable group especially are straight ones.

The compounds of the present invention of the general formula (I) possess activities like lipid A, and therefore are useful as immunity enhancing agents and anti-tumor agents. Concretely, the compounds of the present invention possess the action of activating macrophages, B cell mitogenic activity, producing action of non-specific antibody, enhancing activity of cellular immunity, as blastogenic action, and possess INF (interferon) inducing action, TNF (tumor necrosis factor) including action etc. as anti-tumor action. And it is expected that the compounds also possess producing action of interleukin and stimulating action of NK (natural killer) activity.

The present invention is concerned with all compounds of general formula (I) in the "natural" form or its enantiomeric form, or mixtures thereof, more particularly the racemic form consisting of equimolecular mixtures of natural and its enantiomeric form.

As will be apparent to those skilled in the art, the compounds depicted in general formula (I) have at least five centres of chirality.

That is, when the carbon of 1-, 2-, 3- 4- and 5-position of sugar skeleton and alkyl or alkylene group represented by $R^1$, $R^2$ or $R^3$ are branched-chain, it is possible to occur the centres of chirality.

The presence of chirality leads, as is well know, to the existence of isomerism.

However, all isomers and mixtures thereof which have those side-chains attached to the sugar ring carbon atoms in positions 2 and 4 in the cis-configuration and those side-chains attached to the sugar ring carbon atoms in positions 3 and 5 in the cis-configuration, being such a configuration that the former side-chains (those in position 2 and 4) and the latter side-chains (those in position 3 and 5) are trans with respect to each other and have $R^1$ groups in the 1-position of sugar are to be considered within the scope of the present invention.

## Processes for the Preparation

The present invention is not only chemical compounds per se and non-toxic salts thereof but also processes for the preparation.

According to the present invention, among the compounds of the present invention of the general formula (I), compounds wherein $R^4$ is a hydroxy group, of the general formula:

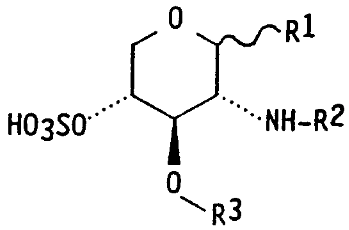

(Ia)

[wherein all of the symbols are the same meaning as defined hereinbefore.]
may be prepared by introducing a sulfo group into hydroxy group of 4-position of the compound of general formula:

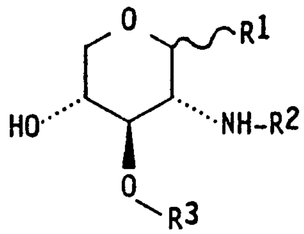

(II)

[wherein all of the symbols are the same meaning as defined hereinbefore.].

This reaction is well known, for example, it may be carried out, in the presence of a tertiary amine (pyridine, triethylamine etc.), in an inert organic solvent (THF, methylene chloride, ethyl acetate etc.) or without solvent used, using with sulfur trioxide-pyridine complex, at a temperature of from -10 °C to 60 °C.

The said reaction using the compound of the general formula (II) wherein $R^1$ is a hydroxy group leads the production of small amounts of by-product in which the sulfoxy groups are introduced at 1-position as well as at the 4-position. The by-product may be removed by purification described hereinafter.

On the other hand, the compounds of the general formula (I) wherein $R^4$ is a sulfoxymethyl group (-$CH_2OSO_3H$), that is, the compound of general formula:

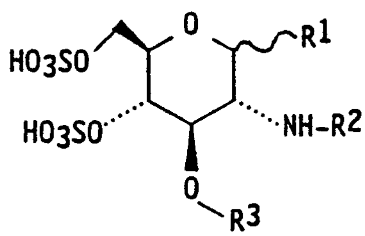

(Ib)

[wherein all of the symbols are the same meaning as defined hereinbefore.]
may be prepared by introducing sulfo groups into hydroxy group of 4- and 6-position of the compound of general formula:

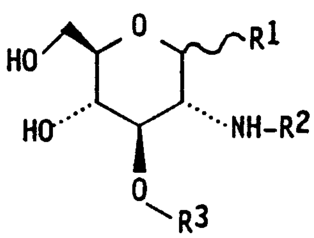

(III)

[wherein all of the symbols are the same meaning as defined hereinbefore.].

This reaction may be carried out by the same method described hereinbefore, by using two-fold amount of sulfur trioxide-pyridine complex required when introducing a sulfo group only into 4-position.

And further, compounds wherein $R^4$ is hydroxymethyl group ($-CH_2OH$); that is, the compound of general formula:

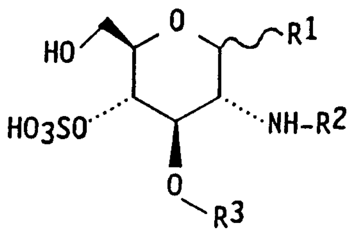

(Ic)

[wherein all of the symbols are the same meaning as defined hereinbefore.] may be prepared by hydrolyzing the compound of general formula:

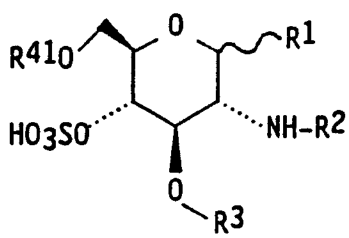

(IV)

[wherein $R^{41}$ represents trialkylsilyl group, the other symbols are the same meaning as defined hereinbefore.] in acidic condition.

Hydrolysis is well known, for example, with using an acid (acetic acid, oxalic acid, trifluoroacetic acid, hydrochloric acid etc.), in a water-miscible organic solvent (THF, methanol, ethanol, dioxane etc.), with or without water, at a temperature of from 0 ° C to 70 ° C.

The compound of the general formula (II) may be prepared by the series of reaction steps described in the following scheme [A], and the compound of the general formula (III) and the compounds of the general formulae (IVa) and (IVb) in those of general formula (IV) may be prepared by the series of reaction steps described in the following scheme [B].

Scheme [A]

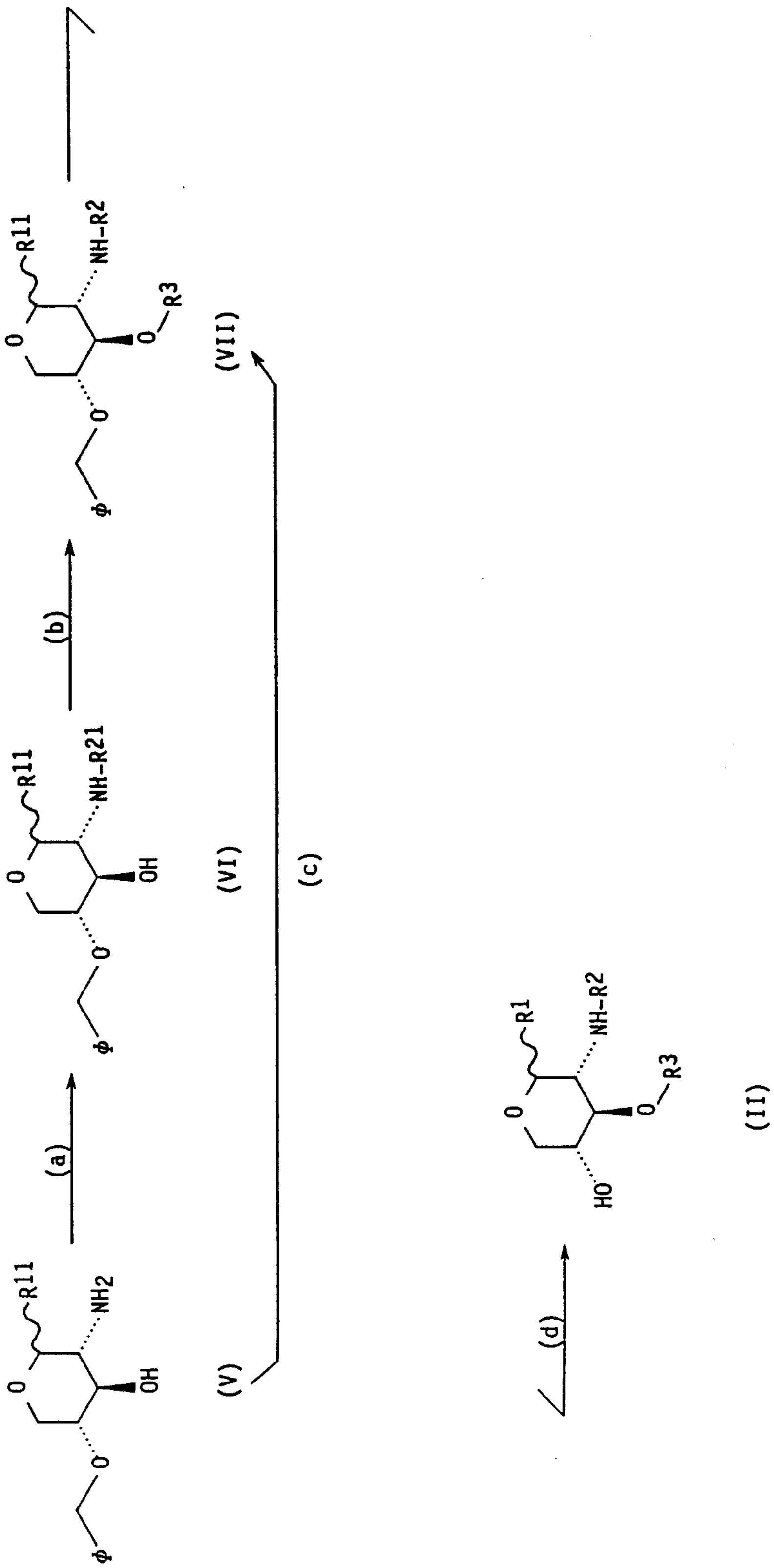


(V)

(VI)

(VII)

(II)

Scheme [B]

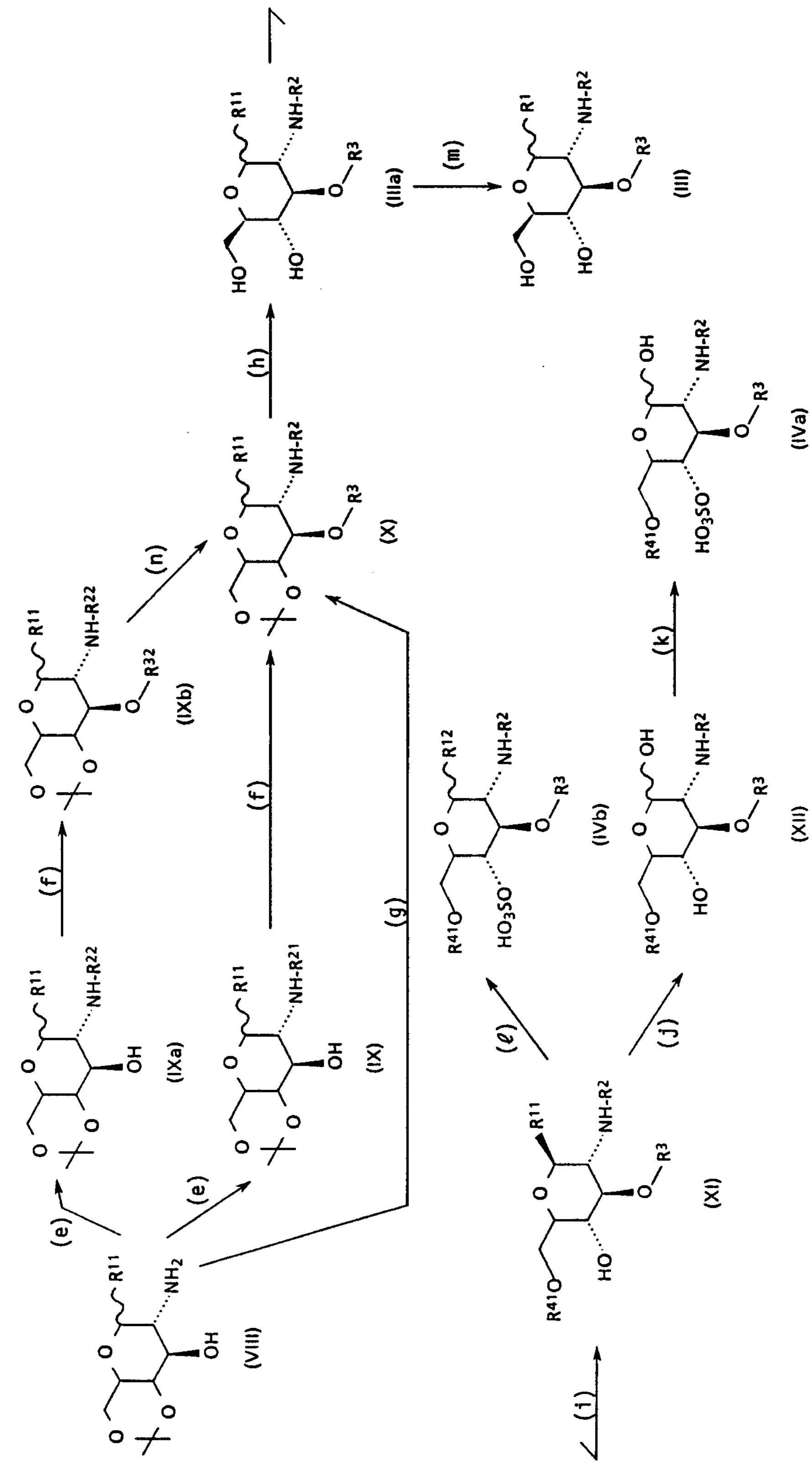


(VIII) (IXa) (IX) (IXb) (X) (IIIa) (III) (XI) (IVb) (XII) (IVa)

Each symbols in the schemes [A] and [B] represent the following meanings or as defined hereinbefore respectively, and the other symbols are same meaning as defined hereinbefore.

$R^{11}$ - hydrogen atom, benzyloxy group or alkoxy group of from 1 to 4 carbon atom(s),

$R^{21}$ - a group of general formula:

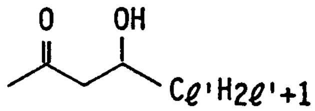 , 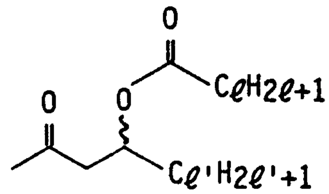 ,

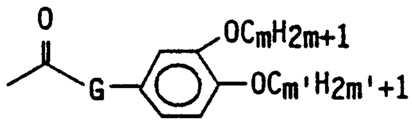 , 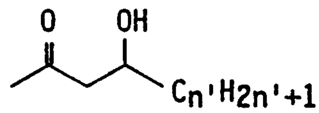 ,

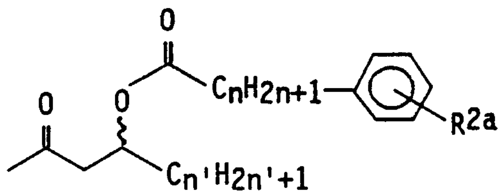 or

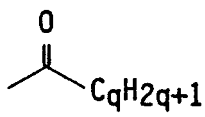

$R^{22}$, $R^{32}$ - being same or different, a group of the general formula:

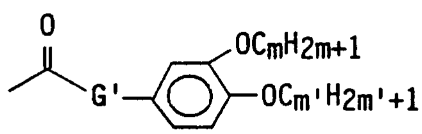 or

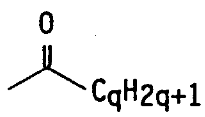 .

With the proviso that, when one group of $R^{22}$ and $R^{32}$ represents a group of the general formula:

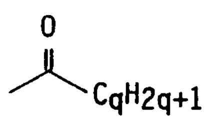 ,

the other group necessarily represents a group of the general formula:

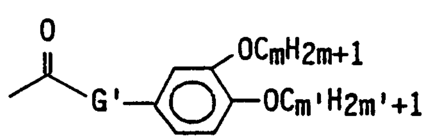 .

G' - alkenylene group of from 2 to 4 carbon atom(s).
$R^{41}$ - trialkylsilyl group,
$R^{12}$ - hydrogen atom or alkoxy group of from 1 to 4 carbon atom(s).

About the reactions (j) and (ℓ) in the scheme [B], the compound of the formula (XI), in which $R^{11}$ is benzoyloxy group may be subjected to the reaction (j), and those in which $R^{11}$ is hydrogen atom or alkoxy group of from 1 to 4 carbon atom(s) may be subjected to the reaction (ℓ).

In the schemes [A] and [B], each reaction steps are known per se, and summarized descriptions are the followings.

Step (a) is an reaction for introducing an acyl group into the amino group, i.e. N-acylation, and it may be carried out, for example, by using a corresponding carboxylic acid of general formula:

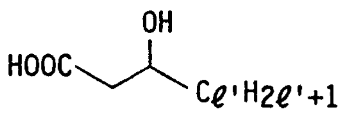

(XIII)

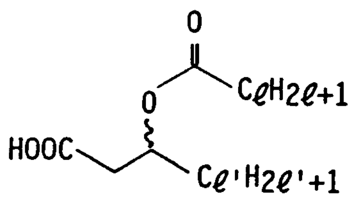

(XIV)

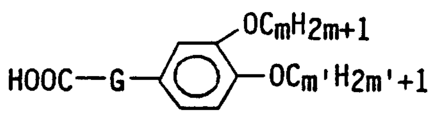

(XV)

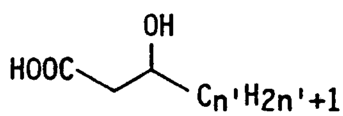

(XVI)

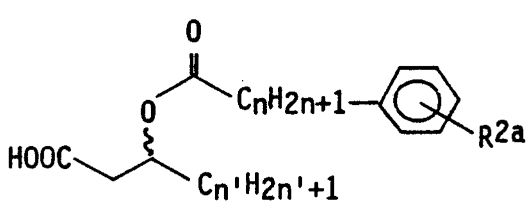

(XVII)

or

HOOC-$C_qH_{2q+1}$ (XVIII)

[wherein all symbols are the same meaning as defined hereinbefore.] and the compound represented by general formula (V), in the presence of a suitable base (triethylamine, pyridine, 4-(N,N-dimethylamino)-pyridine etc.) in an inert organic solvent (methylene chloride, acetonitrile etc.), by using 2-chloro-N-methylpyridinium iodide, at a temperature of from -30 °C to 30 °C.

And, N-acylation described above may also be carried out by the method described hereinafter; that is, by reacting a compound of the general formula (V) and a mixed acid anhydride corresponding to a carboxylic acid of the general formulae (XIII) to (XVIII) at a temperature of from -30 °C to 30 °C. Such anhydride may be obtained by reacting with pivaloyl chloride, in the presence of a suitable base (triethylamine, pyridine etc.), in an inert organic solvent (methylene chloride, toluene, hexane, THF etc.).

And, further, N-acylation also may be carried out by reacting a compound of the general formula (V) and a carboxylic acid of the general formulae (XIII) to (XVIII) in the presence of triphenylphosphine, in an

inert organic solvent (methylene chloride, THF, ethyl acetate etc.), by using 2,2′-pyridyldisulfide, at a temperature of from -10 °C to 50 °C.

And, N-acylation may be carried out by using other methods for activating a carboxylic acid, for example, by using dicyclohexylcarbodiimide.

In such cases, the use of the compound of the general formula (XIII) or (XVI) may produce a compound of the general formula (VII) wherein the same substituents are introduced into the hydroxy groups at the 3-position of the compound of the general formula (XIII) or (XVI) and at the 3-position of the sugar skeleton.

Compounds of the general formula (VII) wherein the above-mentioned two substituents are different each other may be prepared by using the compound of the general formula (XIV) or (XVII).

Step (b) is a reaction for introducing an acyl group into the hydroxy group, i.e. O-acylation, and it may be carried out, for example, in the presence of a suitable base (triethylamine, 4-(N,N-dimethylamino)-pyridine, pyridine etc.), in an inert organic solvent (methylene chloride, toluene, benzene, ethyl acetate, hexane, THF etc.) or without solvent used, by using a corresponding acyl halide, at a temperature of from -10 °C to 60 °C.

And step (b) also may be carried out by reacting with a corresponding carboxylic acid, in the presence of a suitable amine (triethylamine, 4-(N,N-dimethylamino)pyridine, pyridine etc.) in an inert organic solvent (methylene chloride, acetonitrile etc.), by using 2-chloro-N-methylpyridinium iodide as a condencing agent, at a temperature of from -30 °C to 30 °C.

And further, O-acylation may also be carried out by using other methods for activating a carboxylic acid, for example, by using dicyclohexylcarbodiimide.

Step (c) is the acylation of the amino group of 2-position of sugar and the hydroxy group of 3-position of sugar at the same time, and it may be carried out by the same procedure as described in the step (a). This reaction may be carried out by using over two-fold amount of a carboxylic acid of general formulae (XIII) to (XVIII) used in the step (a).

Step (d) is a reaction for removing a benzyl group, and it may be carried out, for example, in an atmosphere of hydrogen, in an organic solvent (methanol, ethanol, THF, THP etc.), by using a hydrogenating catalyst (palladium-carbon, platinum black, nickel etc.), at a temperature of from -10 °C to 80 °C.

In this case, the compound of the general formula (VII) wherein $R^{11}$ is benzyloxy group may be removed benzyl groups of 1- and 4-position of sugar at the same time.

Step (e) is a reaction for introducing an acyl group into the amino group, i.e. N-acylation, and may be carried out by using the compound of the general formula (VIII) by the same procedure as described in the step (a).

Step (f) is a reaction for introducing an acyl group into the hydroxy group, i.e. O-acylation, and may be carried out by the same procedure as described in the step (b).

Step (g) is the acylation of the amino group of 2-position of sugar and the hydroxy group of 3-position of sugar at the same time, and it may be carried out by the same procedure as described in the step (a). This reaction may be carried out by using over two-fold amount of a carboxylic acid of general formulae (XIII) to (XVIII) used in the step (a).

Step (h) is a reaction for removing isopropylidene group on the 4th and 6th positions of the sugar as hydroxy-protecting group, and it may be carried out, for example, in water or a mixed solvent of a water-miscible organic solvent (THF, methanol, ethanol etc.) and water, using an acid (acetic acid, p-toluenesulfonic acid, camphorsulfonic acid, trichloroacetic acid, oxalic acid etc.), at a temperature of from 0 °C to 80 °C.

Step (i) is a reaction for introducing a protecting group into the hydroxy group on the 6-position selectively, and it may be carried out, for example, in the presence of a suitable base (4-(N,N-dimethylamino)pyridine, pyridine, triethylamine etc.), in an inert organic solvent (methylene chloride, toluene, benzene, ethyl acetate, hexane, THF, etc.) or without solvent used, by using a corresponding trialkylsilyl halide (t-butyldimethylsilyl chloride, trimethylsilyl chloride etc.), at a temperature of from -10 °C to 60 °C.

Step (j) is a reaction for removing benzyl group, and it may be carried out by the same procedure as described in the step (d).

Step (k) is a reaction for introducing an sulfo group into the hydroxy group on the 4-position of sugar, and it may be carried out, for example, in the presence of a tertiary amine (pyridine, triethylamine etc.), in an inert organic solvent (THF, methylene chloride, ethyl acetate etc.) or without solvent used, using sulfur trioxide-pyridine complex, at a temperature of from -10 °C to 60 °C.

Step (ℓ) is a reaction for introducing a sulfo group into the hydroxy group on the 4-position of sugar, and it may be carried out by the same procedure as described in the step (k).

Step (m) is a reaction for removing benzyl group in the compound of general formula (IIIa) wherein $R^{11}$ is benzyloxy group, and it may be carried out by the same procedure as described din the step (d).

Step (n) is a catalytic reduction, and it may be carried out by the same procedure as described in the step (d).

Throughout the specification, in each reactions, products may be purified by conventional methods, for example, distillation at atmospheric or reduced pressure, high performance liquid chromatography, thin layer chromatography using silica gel or magnesium silicate or washing or recrystallization. Purification may be carried after each reactions or a series of reactions.

## Starting materials

Starting materials and reagents using in the present invention are known compounds per se or may be prepared by known methods per se.

For example, the compound of the general formula (VIII) wherein $R^{11}$ is benzyloxy group is reported in Agric. Biol. Chem., 48(1), 251 (1984).

The compound of the general formula (V) wherein $R^{11}$ is hydrogen atom, may be synthesized easily from the compound described in J. Org. Chem., 30(4), 1282 (1965) by known methods.

## Salts

The compounds of the present invention of the general formula (I) may be formed salts at the sulfo group.

By converting into salts, solubility of the compounds of the present invention against water can be increased, and therefore be useful for the administration as pharmaceuticals.

The compounds of the present invention may easily be converted into corresponding salts by the methods known per se, e.g. methods described hereafter.

The salts in the present invention are preferably non-toxic ones. The non-toxic salts herein referred mean salts of cations such that it is relatively innoxious to living body (animals including human beings) tissues and that the effective pharmacological properties of the compounds of the general formula (I) are not impaired by side effect(s) resulting from the cations when used in an amount required for the prevention and/or treatment. And water-soluble salts are preferable.

Suitable salts include, for example, a salt of an alkali metal such as sodium, potassium, a salt of an alkaline earth metal such as calcium, magnesium, an ammonium salt and a pharmaceutically acceptable (non-toxic) amine salt.

Amines suitable for forming such salts with sulfo group are well known and include, for example, those amines which are theoretically obtained by substituting one or more of hydrogen atom(s) of ammonia by other group(s). These groups, which may be the same or different when one or more hydrogen atom(s) are substituted, are selected from, for example, alkyl group(s) of from 1 to 6 carbon atom(s) and hydroxyalkyl group(s) of from 1 to 3 carbon atom(s). Suitable non-toxic amine salts include salts of a tetraalkylammonium group, such as tetramethylammonium salt and salts of an organic amine, such as methylamine, dimethylamine, triethylamine, cyclopentylamine, benzylamine, phenetylamine, pyridine, piperidine, monoethanolamine, diethanolamine, lysine and alginine.

Salts can be obtained from the compounds of the present invention of the general formula (I), by methods known per se, for example, by reacting the compound of the general formula (I) and a suitable base such as a hydroxide or carbonate of an alkali metal or alkaline earth metal, ammonium hydroxide, ammonia or an organic amine in theoretical amounts in an appropriate solvent.

The salts can be isolated by freeze-drying the solution, or by filtration if the salts are sufficiently insoluble to the reaction solution, of if necessary, by removing part of the solvent followed by filtration.

## Pharmacological activities

The compounds of the present invention of the general formula (I) possess lipid A like activities described hereinbefore, for example, in a standard laboratory test, results in the followings are given.

### (1) Mitogenic activity in vitro

The compound of the present invention showed activities as in the following Table I, with the test system described hereafter.

Table I

| Example No. of the compounds | Blastgenic Index |
|---|---|
| 1 (b) | 13.7 |
| 1 (c) | 13.8 |
| 1 (g) | 17.6 |
| 2 | 27.7 |

Mitogenic activity means the level of mitogenesia of lymph cells, and may be shown as "Blastogenic Index" by measuring the amount of tymidine which is taken into lymph cells.

For example, the value of "13.7" in the top of Table I means that 13.7 times amount of tymidine can be taken into lymph cells by adding the compound prepared in example 1(b) to the test system in comparison with no addition of the test compound.

The amounts were measured by dencity of radiolabel.

Mitogenic activity in vitro was measured by the following method (See Procedure Method of Immunoexperiment pp 315 published by Japan Immunology Society).

C3H/He male mice of 6 weeks old were killed by bloodletting, and spleens were isolated and homogenized. Homogenizing liquids were filtered with gauze, and suspending cells were prepared with PBS solution. After washing, lymphocytes fractions were gathered with lympholight M. Lymphocytes were suspended in RPMI 1640 cultivation solution (10% FEBS), and the solution was divided into 5 x $10^5$ cells/180 $\mu\ell$/wells. Ten times concentration of the terminal concentration (1 $\mu$g/m$\ell$) of the compounds of the present invention were added with 20 $\mu\ell$ portions to the each wells.

In an atmosphere of a mixed gas of 5% $CO_2$ - 95% $O_2$, the cells were cultivated for 24 hrs at 37°C. After cultivation, $^3$H-tymidine 0.5 $\mu$Ci/20 $\mu\ell$ was added to the each wells. And more 24 hrs cultivation was continued at 37°C, and taking amounts of $^3$H-tymidine into the cells were measured. Mitogenic activities were showed by Blastogenic index in the followings.

$$\text{Blastgenic index} = \frac{\text{Taking amount of } ^3\text{H-tymidine into the lymphocytes with adding the compound of the present invention}}{\text{Taking amount of } ^3\text{H-tymidine into the blank control lymphocytes}}$$

(2) Inducing activities of TNF in vitro

The compounds of the present invention showed activities as in the following Table II, with the test system described hereafter.

Table II

| Example No. of the compounds | Cytotoxicities (%) |
|---|---|
| 1 | 50.7 |
| 1 (b) | 30.2 |
| 1 (g) | 87.9* |
| 2 | 20.0 |

* serum dilution 1/25

Inducing activity of TNF in vitro was measured by the following test system.

Coryne parvum (manufactured by Ribi Immuno Chem. Research Inc.) was administered to ICR male mice of 6-week old from tail vein as priming agent. Ten days' after, the compounds of the present invention (10 μg) were administered from tail vein as eliciting agent. 90 mins' after of the administration, bloods were collected from heart, and blood serums were separated.

Mice L-M cells ($10^4$/100 μℓ/well) suspended in a mixture of the diluted serum solution (100 μℓ/well) and RPMI 1640 cultivating solution (10% FEBS and 0.2% glucose; manufactured by Nissui Pharmaceutical Co.) were cultivated in an atmosphere of a mixed gas of 5% $CO_2$ - 95% $O_2$, for 48 hrs at 37 °C, after adding $^3$H-tymidine (0.5 μCi/20 μℓ/well). Taking amounts of $^3$H-tymidine into cells during the cultivation were measured.

Inducing activities of TNF were showed by cytotoxicity in the followings.

$$\text{Cytotoxicity (\%)} = \left(1 - \frac{\text{Taking amount of } {}^3\text{H-tymidine into the lymphocytes with adding the compound of the present invention}}{\text{Taking amount of } {}^3\text{H-tymidine into the blank control lymphocytes}}\right) \times 100$$

Toxicity

On the other hand, it was confirmed that the toxicity of the compounds of the present invention were very low. Therefore, the compounds of the present invention may be considered to be sufficiently safe and suitable for pharmaceutical used.

Application for Pharmaceuticals

In mammals including human beings, especially human beings, decrease of immunity by ageing and disorders including immuno deficiency induce fatal infections e.g. opportunistic infection.

The compounds of the present invention of the general formula (I) possess enhancing activity of cellular immunity (e.g. mitogenic activity) to living tissue as shown in the experiment hereinbefore, and therefore be useful for enhancing agent of immunity.

And, the compounds of the present invention of the general formula (I) possess and inducing activity of TNF, an inducing activity of IL-1 and an inducing activity of IFN, and therefore be useful for anti-tumor agent.

For the purpose hereinbefore described, the compounds of the present invention of the general formula (I) or non-toxic salts thereof may normally be administered systemically or partially; usually by oral or parenteral administration.

The doses to be administered are determined depending upon age, body weight, symptom, the desired therapeutic effect, the route of administration, and the duration of the treatment etc. In the human adult, the doses per person per dose are generally between 0.1 mg and 100 mg, by oral administration, up to several times per day, and between 10 µg and 10 mg, by parenteral administration up to several times per day.

As mentioned above, the doses to be used depend upon various conditions. Therefore, there are cases in which doses lower than or greater than the ranges specified above may be used.

Solid compositions according to the present invention for oral administration include compressed tablets, dispersible powders and granules. In such solid compositions, one or more of the active compound(s) is or are, admixed with at least one inert diluent such as lactose, mannitol, glucose, hydroxypropylcellulose, microcrystalline cellulose, starch, polyvinylpyrrolidone or magnesium metasilicate aluminate. The compositions may also comprise, as is normal practice, additional substances other than inert diluents e.g. lubricating agents such as magnesium stearate, disintegrating agents such as cellulose calcium gluconate, and assistant for dissolving e.g. arginine, glutamic acid or amino-acid such as aspartic acid. The tablets or pills may, if desired, be made into gastric film-coated or enteric film-coated tablets or pills, such as sugar-coated, gelatin-coated, hydroxypropyl cellulose-coated or hydroxypropylmethyl cellulose phthalate-coated tablets or pills and, two or more of layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and preserving agents.

Other compositions for oral administration include spray compositions which may be prepared by known methods and which comprise one or more of the active compound(s).

Preparations for injection according to the present invention for parenteral administration include sterile aqueous or non-aqueous solutions, suspensions or emulsions. Example of aqueous solvents or suspending media are distilled water for injection and physiological salt solution. Examples of non-aqueous solvents or suspending media are propylene glycol, polyethylene glycol, vegetable oils such as olive oil, alcohols such as ethanol, POLYSORBATE 80 (registered Trade Mark). These compositions may also include adjuvants such as preserving, wetting, emulsifying, dispersing agents and assistant agent for dissolving (e.g. arginine, glutamic acid or amino-acid such as aspartic acid). They may be sterilized, for example, by filtration through a bacteria-retaining filter, by incorporation of sterilizing agents in the compositions or by irradiation. They may also be manufactured in the form of sterile solid compositions which can be dissolved in sterile water or some other sterile injectable medium immediately before use.

Other compositions for parenteral administration include liquids for external use, and endermic liniments such as ointments, suppositories and pessaries which comprise one or more of the active compound(s) and may be prepared by known methods.

## Nomenclature

Throughout the specification including claims, the compounds of the present invention and named as derivatives of glucopyranose having the following structure.

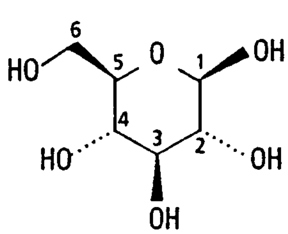

≡

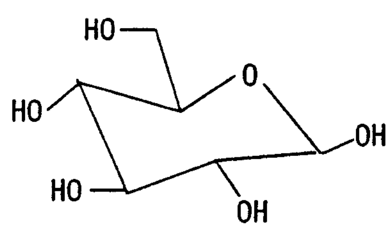

In the structural formula of the specification including claims, the dotted line (---), the thickened line ( ) and the wavy line ( ) indicate that the respective group attached thereto is in the backside of the plane, i.e. in α-configulation, in the front of the plane, i.e. in β-configulation, and in α- or β-configulation or a mixture

thereof, respectively, according to the generally accepted nomenclatural rule.

Reference examples and Examples

The following reference examples and examples are illustrate the present invention, but not limit the present invention.

In the reference examples and examples, "TLC" and "IR" represent "Thin layer chromatography" and "Infrared absorption spectrum", respectively.

The solvents in the parentheses show the developing or eluting solvents and the ratios of the solvents used are by volume in chromatographic separations.

Unless otherwise specified, "IR" was measured by KBr tablet method.

Reference example 1

Synthesis of 2-deoxy-2-(3-hydroxytetradecanoyl)amino-4-O-benzyl-1,5-anhydro-D-xylitol

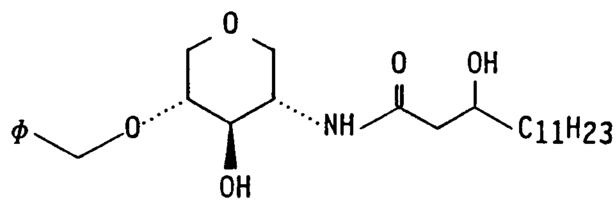

The solution of ethyl acetate of 2,2'-dipyridyldisulfide was added slowly to the suspension of 2-deoxy-2-amino-4-O-benzyl-1,5-anhydro-D-xylitol (1.07 g), triphenylphosphine (3.02 g) and 3-hydroxymiristic acid (1.23 g).

The mixture was stirred for 5 hours at room temperature, and then the reaction solution was washed with successively, water, 1N aqueous solution of sodium hydroxide and a saturated aqueous solution of sodium chloride, and dried and evaporated.

The residue was purified by column chromatography on silica gel ($CH_2C\ell_2$:AcOEt = 1:1) to give the title compounds (1.74 g) having the following physical data.

TLC: Rf 0.33 ($CH_2C\ell_2$:MeOH - 1:20);

IR : $\nu$ 3400, 3280, 2900, 2840, 1730, 1650, 1460 $cm^{-1}$.

Reference example 1(a) ~ 1(b)

By the same procedure as reference example 1, following compounds having the physical data shown in the Table III were given.

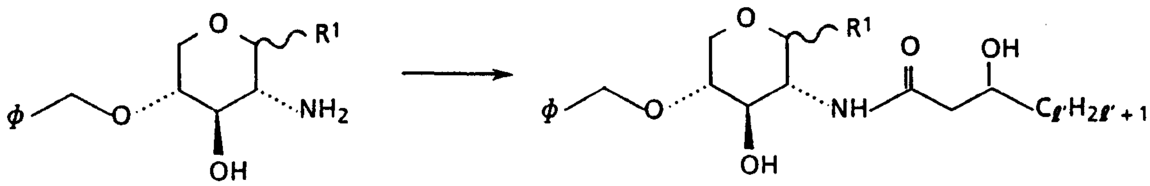

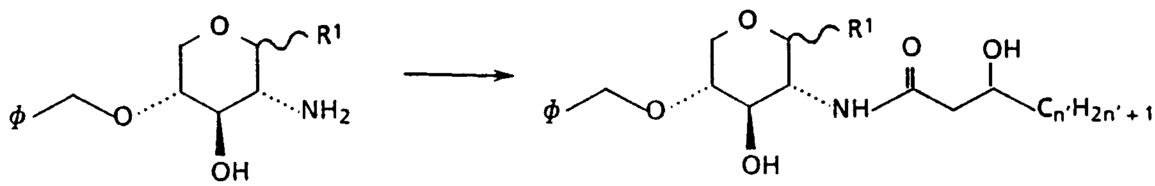

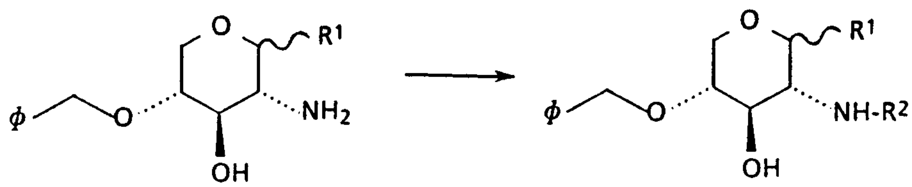

Table III

| No. | $R^1$, $R^2$ | Name | TLC | IR ($cm^{-1}$) |
|---|---|---|---|---|
| 1 (a) | R1: —H<br>R2: 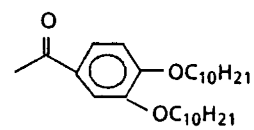 | 2-deoxy-2-(3,4-didecyloxybenzoyl)amino-4-O-benzyl-1,5-anhydro-D-xylitol | 0.42 (AcOEt:n-hexane 1:1) | ν 3400, 3260, 2910, 2840, 1615, 1595, 1575, 1500, 1455, 1260, 1215, 1090 |
| 1 (b) | R1: ·······$OCH_3$<br>R2: 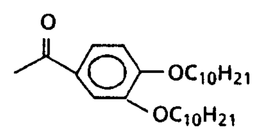 | Methyl 2-deoxy-2-(3,4-didecyloxybenzoyl)amino-4-O-benzyl-1,5-anhydro-α-D-xyloside | | |

Reference example 2

Synthesis of 2-deoxy-2-[3-(9-phenylnonanoyl)oxytetradecanoyl]amino-3-O-(9-phenylnonanoyl)-4-O-benzyl-1,5-anhydro-D-xylitol

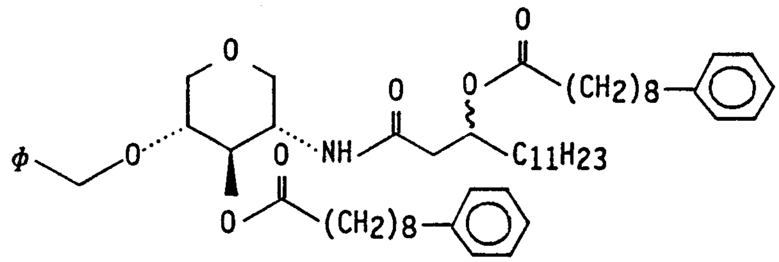

Triethylamine (0.47 mℓ) was added to the suspension of compound (500 mg) prepared in reference example 1, phenylnonanoic acid (635 mg) and 2-chloro-1-methyl-pyridiniumiodide (710 mg) in methylene chloride (15 mℓ), and further 4-(N,N′-dimethylamino)pyridine (135 mg) was added thereto, and the mixture was reacted overnight at room temperature.

The reaction solution was diluted with ethyl acetate, washed with successively, water, 1N aqueous solution of sodium hydroxide and a saturated aqueous solution of sodium chloride, dried and evaporated. The residue was purified by column chromatography on silica gel (n-hexane:AcOEt = 1:1), and further twice purification using column chromatography on the same condition was carried out to give the title compounds (more polar: 261 mg) having the following physical data.

TLC: Rf 0.26 (AcOEt:n-hexane = 1:2).

Reference example 2(a) ~ 2(f)

By the same procedure as reference example 2, using each starting material, following compounds having the physical data shown in the Table IVa and IVb were given.

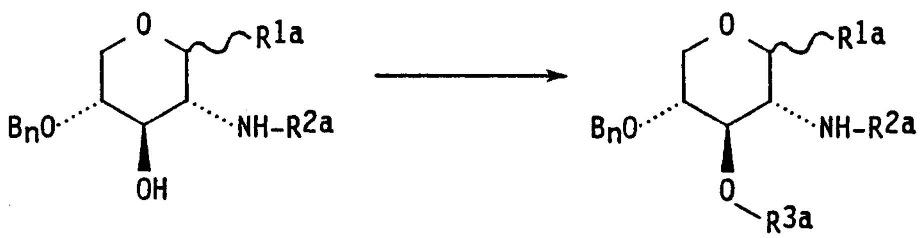

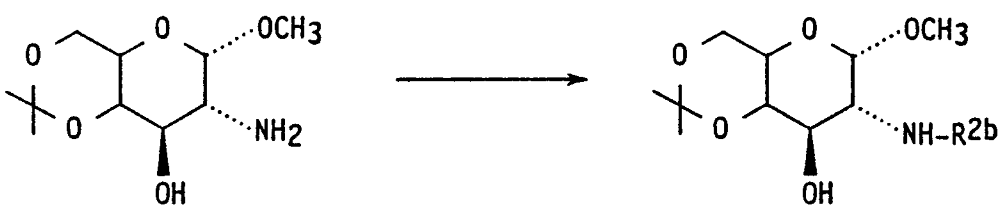

Table IVa

| No. | R1a, R2a, R3a | Name | Starting material | TLC | IR ($cm^{-1}$) |
|---|---|---|---|---|---|
| 2 (a) | R1a: —H<br>R2a:<br>R3a:<br>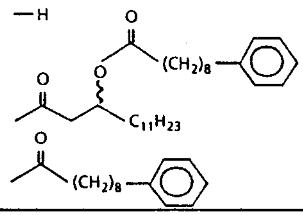 | 2-deoxy-2-[3-(9-phenylnonanoyl)oxytetradecanoyl]amino-3-O-(9-phenylnonanoyl)-4-O-benzyl-1,5-anhydro-D-xylitol (less polar) | reference example 1 | 0.36 (AcOEt:n-hexane = 1:2) | |
| 2 (b) | R1a: —H<br>R2a:<br>R3a:<br>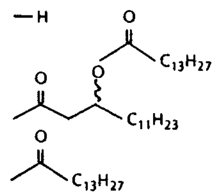 | 2-deoxy-2-[3-tetradecanoyloxytetradecanoyl]amino-3-O-tetradecanoyl-4-O-benzyl-1,5-anhydro-D-xylitol (less polar) | reference example 1 | 0.69 (AcOEt:n-hexane = 3:5) | ν 3290, 2900, 2820, 1720, 1650, 1520 |
| 2 (c) | R1a: —H<br>R2a:<br>R3a:<br>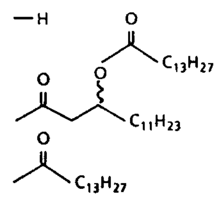 | 2-deoxy-2-[3-tetradecanoyloxytetradecanoyl]amino-30-tetradecanoyl-4-O-benzyl-1,5-anhydro-D-xylitol (more polar) | reference example 1 | 0.65 (AcOEt:n-hexane = 3:5) | ν 3380, 2900, 283C, 1720, 1710, 1640, 1540 |

Table IVa (continued)

| | | | | | |
|---|---|---|---|---|---|
| 2 (d) | R1a: 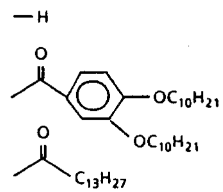<br>R2a:<br>R3a: | 2-deoxy-2-(3,4-didecyloxybenzoyl)amino-3-O-tetradecanoyl-4-O-benzyl-1,5-anhydro-D-xylitol | reference example 1 (a) | 0.63 (AcOEt:n-hexane: chloroform = 1:4:1) | ν 3350, 2900, 2840, 1720, 1625, 1535, 1505, 1455, 1340, 1270, 1215 |
| 2 (e) | R1a: 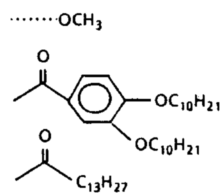<br>R2a:<br>R3a: | Methyl 2-deoxy-2-(3,4-didecyloxybenzoyl)amino-3-O-tetradecanoyl-4-O-benzyl-1,5-anhydro-α-D-xyloside | reference example 1 (b) | | |

Table IVb

| No. | $R^{2b}$ | Name | TLC | IR ($cm^{-1}$) |
| --- | --- | --- | --- | --- |
| 2 (f) | $R^{2b}$: 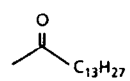 | methyl 2-deoxy-2-tetradecanoyl-4,6-O-isopropilydene-α-D-glucopyranoside | 0.75 (MeOH:$CH_2Cl_2$ = 3:20) | ν 3450, 3320, 2920, 2850, 1640, 1530, 1460 |

Reference example 3

Synthesis of benzyl 2-deoxy-2-(3,4-didecyloxybenzoyl)amino-4,6-O-isopropylidene-$\beta$-D-glucopyranoside

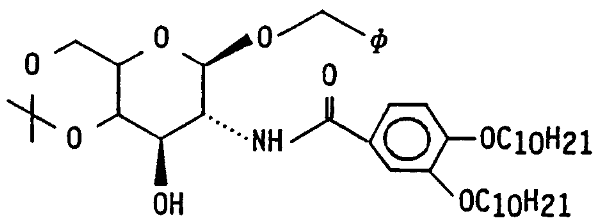

$\alpha,\alpha'$-dipyridyl disulfide (330 mg) was added to a solution of benzyl 2-deoxy-2-amino-4,6-O-isopropylidene-$\beta$-D-glucopyranoside (the compound described in Agric. Biol. Chem., 48(1), 251 (1984); 309 mg), (3,4-didecyloxy)carboxylic acid (521 mg) and triphenylphosphine (393 mg) in ethyl acetate (10 mℓ), and stirred for 2 hours at room temperature, and further stirred for 2 hours at 50 ° C. After the reaction, the solution was stirred overnight at room temperature. 4-(N,N-dimethylamino)pyridine (244 mg) was added thereto, and the mixture was stirred for 5 hours at room temperature.

The reaction mixture was diluted with the mixture of hexane-ethyl acetate (n-hexane:EtOAc = 1:1; 50 mℓ), and washed with successively, 1N aqueous solution of sodium hydroxide, water 1N-hydrochloric acid, water and a saturated aqueous solution of sodium chloride, and then dried and evaporated.

The residue was purified by column chromatography on silica gel ($CH_2C\ell_2$:EtOAc = 7:3) to give the title compounds (612 mg) having the following physical data.

TLC: Rf 0.63 (EtOAc);

IR : $\nu$ 3520, 3350, 2940, 2860, 1640, 1600, 1592, 1530, 1510, 1475, 1263, 1220, 1113, 1090, 1036, 1010, 853, 804, 758, 728, 690 $cm^{-1}$.

Reference example 3(a)

Synthesis of benzyl 2-deoxy-2-tetradecanoylamino-4,6-O-isopropylidene-$\beta$-D-glucopyranoside

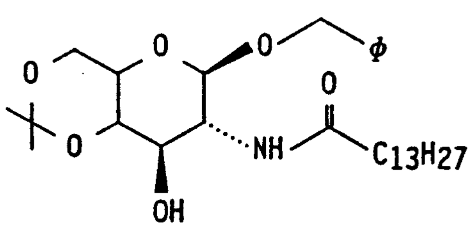

By the same procedure as reference example 3, the title compound (510 mg) having the following physical data was obtained by using the same starting material.

TLC: Rf 0.45 (AcOEt:n-hexane = 3:1);

IR : $\nu$ 3500, 3275, 2400, 2340, 1635, 1540, 1460, 1370, 1265, 1195, 1080 $cm^{-1}$.

Reference example 3(b)

Synthesis of benzyl 2-deoxy-2-[3-(3,4-dioctyloxy)phenylpropionyl]amino-4,6-O-isopropylidene-β-D-glucopyranoside

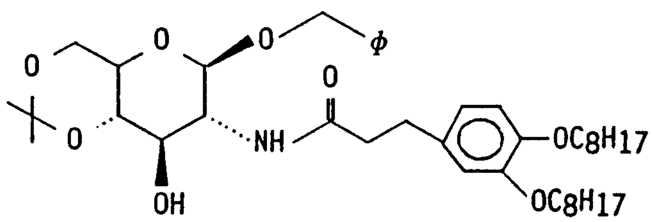

By the same procedure as reference example 3, the title compound (1.21 g) having the following physical data was obtained by using the same starting material.

TLC: Rf 0.67 (AcOEt:n-hexane = 3:1);

IR : ν 3950, 2910, 2895, 1665, 1585, 1530, 1510, 1260, 1090 $cm^{-1}$.

Reference example 4

Synthesis of methyl 2-deoxy-2-(3,4-didecyloxybenzoyl)amino-4,6-O-isopropylidene-α-D-glucopyranoside

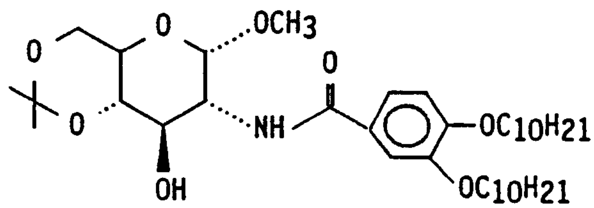

Methyl 2-deoxy-2-amino-4,6-O-isopropyridene-β-D-glucopyranoside (0.5 g), 3,4-di(decyloxy)carboxylic acid (1.21 g) and 1-methyl-2-chloropyridinium iodide (0.93 g) was suspended in methylene chloride, and triethylamine (0.68 mℓ) was added at room temperature and the mixture reacted overnight.

The reaction solution was diluted with methylene chloride, and then was washed with successively a saturated aqueous solution of sodium bicarbonate and a saturated aqueous solution of sodium chloride, dried and evaporated.

The residue was purified by column chromatography on silica gel ($CH_2Cℓ_2$:n-hexane:AcOEt = 1:1:4) to give the title compounds (1.0 g) having the following physical data.

TLC: Rf 0.18 (AcOEt:n-hexane = 1:2).

Reference example 4(a)

Synthesis of methyl 2-deoxy-2-[3-(3,4-dioctyloxyphenyl)propenyl]amino-4,6-O-isopropylidene-α-D-glucopyranoside

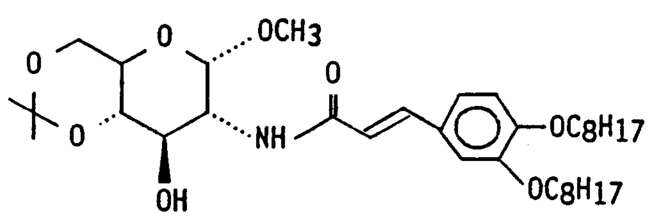

By the same procedure as reference example 4, the title compound having the following physical data was obtained by using same starting material.

TLC: Rf 0.75 ($CH_2Cl_2$:MeOH = 9:1);

IR : ν 3350, 3280, 2920, 2850, 1650, 1610, 1600, 1510 $cm^{-1}$.

Reference example 5

Synthesis of benzyl-2-deoxy-2-(3,4-didecyloxybenzoyl)amino-3-O-(3,4-didecyloxybenzoyl)-4,6-O-isopropylidene-β-D-glucopyranoside

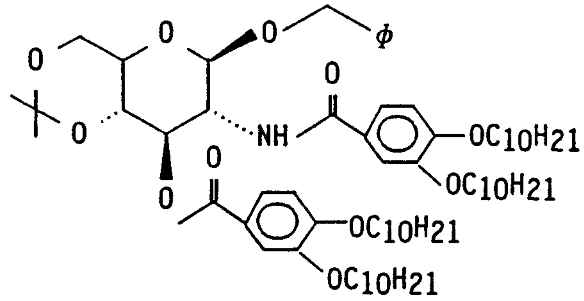

Benzyl 2-deoxy-2-amino-4,6-O-isopropylidene-β-D-glucopyranoside was used as starting material, and the title compound (290 mg) having the following physical data was obtained by the same procedure as reference example 2.

TLC: Rf 0.8 (AcOEt:n-hexane = 3:5);

IR : ν 3270, 2930, 2850, 1720, 1640, 1600, 1540, 1510, 1460 $cm^{-1}$.

Reference example 5(a) ~ 5(c)

By the same procedure as reference example 2, following compounds having the physical data shown in the Table V were given.

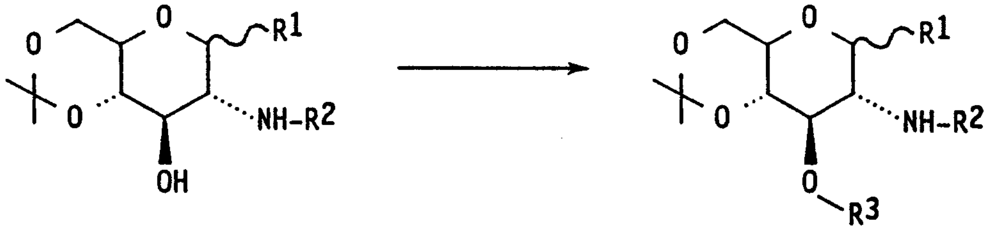

Table V

| No. | R1, R2, R3 | Name | Starting material | TLC | IR (cm-1) |
|---|---|---|---|---|---|
| 5 (a) | R1: 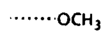 R2,R3: 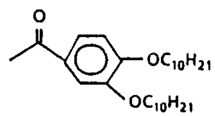 | Methyl 2-deoxy-2-(3,4-didecyloxybenzoyl)amino-3-O-(3,4-didecyloxybenzoyl)-4,6-O-isopropylidene-α-D-glucopyranoside | reference example 4 | 0.56 (AcOEt:n-hexane = 1:2) | ν 3300, 2910, 1710, 1620, 1590, 1570, 1500, 1460, 1420, 1370, 1330, 1270, 1210, 1120, 990 |
| 5 (b) | R1: 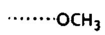 R2,R3: 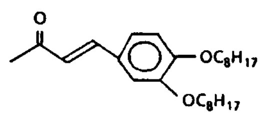 | Methyl 2-deoxy-2-[3-(3,4-dioctyloxyphenyl)propenoyl]amino-3-O-[3-(3,4-dioctyloxyphenyl)propenoyl]4,6-O-isopropylidene-α-D-glucopyranoside | reference example 4 (a) | 0.95 (AcOEt:$CH_2C\ell_2$ = 1:5) | ν 3350, 2900, 2850, 1700, 1650, 1620, 1590, 1510, 1460 |
| 5 (c) | R1: 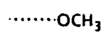 R2: 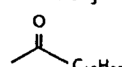 R3: 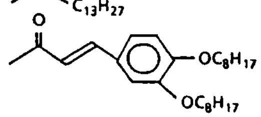 | Methyl 2-deoxy-2-tetradecanoylamino-3-[3-(3,4-dioctyloxyphenyl)propenoyl]-4,6-O-isopropylidene-α-D-glucopyranoside | reference example 2 (f) | 0.80 (AcOEt:$CH_2C\ell_2$ = 1:3) | ν 3270, 2930, 285C, 1710, 1640, 151C |

Reference example 6

Synthesis of benzyl 2-deoxy-2-(3,4-didecyloxybenzoyl)amino-3-O-tetradecanoyl-4,6-O-isopropylidene-β-D-glucopyranoside

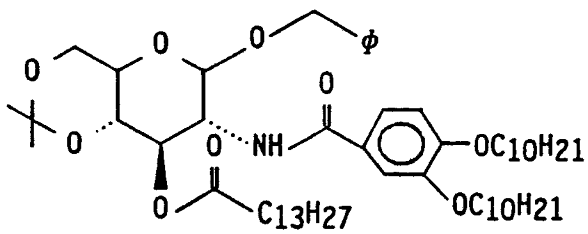

Tetradecanoyl chloride (0.27 mℓ) was added to a solution of the compound (600 mg) prepared in reference example 3 and pyridine (0.16 mℓ) in methylene chloride at room temperature. After the mixture was stirred for two hours, methylene chloride (40 mℓ) was added to this reaction solution.

The reaction solution was washed with successively, 1N-hydrochloric acid and water, dried and evaporated to give the title compounds having the following physical data.

TLC: Rf 0.64 ($CH_2Cℓ_2$:EtOAc = 10:1).

Reference example 6(a) ~ 6(d)

By the same procedure as reference example 6, using each starting material, following compounds having the physical data shown in the Table VI were given.

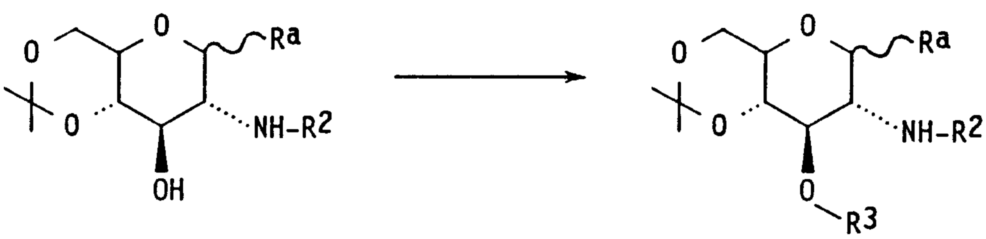

Table VI

| No. | R2, R3, Ra | Name | Starting material | TLC | IR (cm-1) |
|---|---|---|---|---|---|
| 6 (a) | R2: 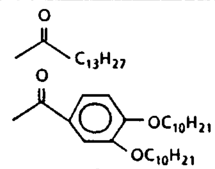<br>R3:<br>Ra: 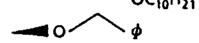 | Benzyl 2-deoxy-2-tetradecanoylamino-3-O-(3,4-didecyloxybenzoyl)-4,6-O-isopropylidene-β-D-glucopyranoside | reference example 3 (a) | 0.72 (AcOEt: chloroform = 1:10) | ν 3340, 2900, 2840, 1705, 1645, 1590, 1510, 1455, 1420, 1260, 1195, 1070 |
| 6 (b) | R2: 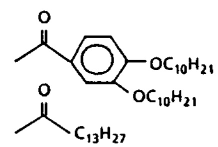<br>R3:<br>Ra: 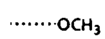 | Methyl 2-deoxy-2-(3,4-didecyloxybenzoyl)amino-3-O-tetradecanoyl-4,6-O-isopropylidene-α-D-glucopyranoside | reference example 4 | 0.49 (AcOEt:n-hexane = 1:2) | |
| 6 (c) | R2: 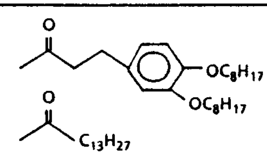<br>R3:<br>Ra: 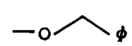 | Benzyl 2-deoxy-2-[3-(3,4-dioctyloxy)phenylpropionyl]amino-3-O-tetradecanoyl-4,6-O-isopropylidene-β-D-glucopyranoside | reference example 3 (b) | 0.49 (AcOEt:n-hexane = 1:3) | ν 3350, 2910, 2845, 1710, 1650, 1520, 1470, 1255, 1240 |

Table VI (continued)

| | | | | | |
|---|---|---|---|---|---|
| 6 (d) | Ra:<br>R2:<br>R3: 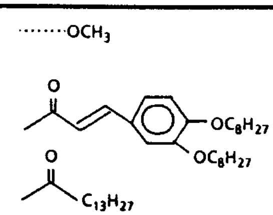 | Methyl 2-deoxy-2-[3-(3,4-dioctyloxyphenyl)propenyl]amino-3-O-tetradecanoyl-4,6-O-isopropylidene-α-D-glucopyranoside | reference example 4 (a) | 0.81 (AcOEt:$CH_2C\ell_2$ = 1:5) | ν 3370, 2930, 2850, 1730, 1660, 1620, 1600, 1510 |

Reference example 6-1

Synthesis of methyl 2-deoxy-2-[3-(3,4-dioctyloxyphenyl)propanoyl]amino-3-O-tetradecanoyl-4,6-O-isopropylidene-α-D-glucopyranoside

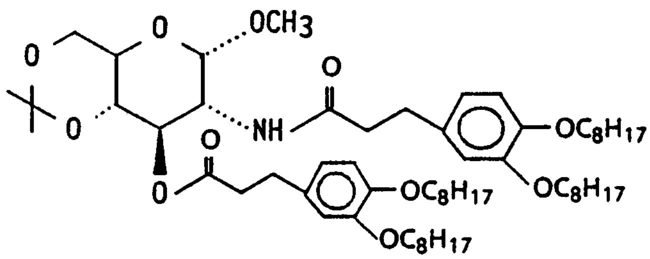

10% Pd-c (0.2 g) was added to a solution of the compound (1 g) prepared in reference example 5(b), of THF (20 mℓ). Under an atmosphere of hydrogen, the mixture was stirred overnight at room temperature.

After the reaction, the catalyst was filtered off, and the filtrate was evaporated to give the title compounds (0.98 g) having the following physical data.

TLC: Rf 0.61 (AcOEt:n-hexane:$CH_2Cℓ_2$ = 1:3:1);

IR : ν 3270, 2910, 2850, 1730, 1650, 1510, 1460 $cm^{-1}$.

Reference example 6-1(a) ~ 6-1(b)

By the same procedure as reference example 6-1, using each starting material, following compounds having the physical data shown in the Table VII were given.

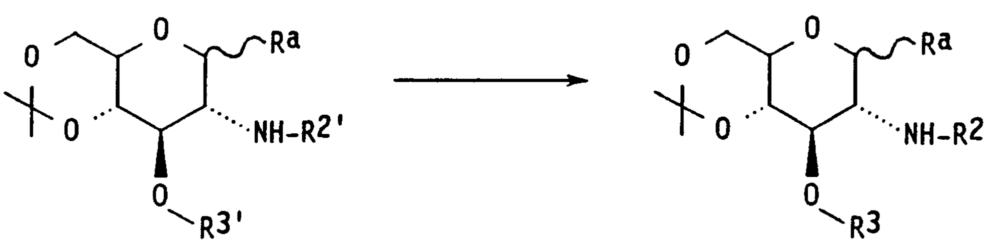

Table VII

| No. | Ra, R2', R3', R2, R3 | Name | Starting material | TLC | IR (cm-1) |
|---|---|---|---|---|---|
| 6-1 (a) | Ra :<br>R2' :<br>R3' :<br>R2 :<br>R3 : 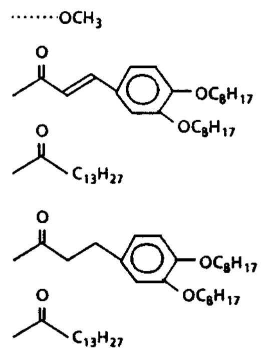 | Methyl 2-deoxy-2-[3-(3,4-dioctyloxyphenyl)propanoyl]amino-3-O-tetradecanoyl-4,6-O-isopropylidene-α-D-glucopyranoside | reference example 6 (d) | 0.61 (AcOEt:$CH_2Cl_2$:n-hexane = 1:1:3) | ν 3370, 2920, 2850, 1730, 1660, 1510, 1460 |
| 6-1 (b) | Ra :<br>R2' :<br>R3' :<br>R2 :<br>R3 : 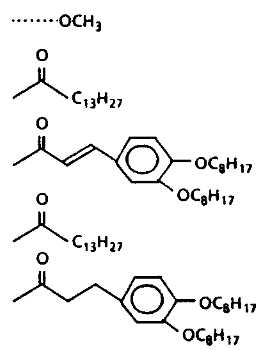 | Methyl 2-deoxy-2-tetradecanoylamono-3-O-[3-(3,4-dioctyloxyphenyl)propanoyl]-4,6-O-isopropylidene-α-D-glucopyranoside | reference example 5 (c) | 0.61 (AcOEt:$CH_2Cl_2$:n-hexane = 1:1:3) | ν 3300, 2910, 2850, 1730, 1640, 1510 |

Reference example 7

Synthesis of benzyl 2-deoxy-2-(3,4-didecyloxybenzoyl)amino-3-O-tetradecanoyl-β-D-glucopyranoside

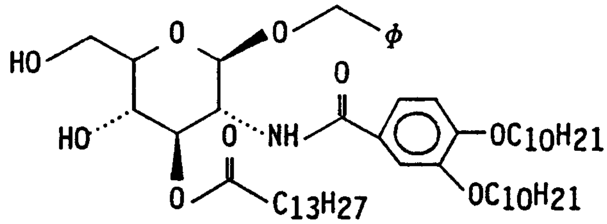

Water (4 mℓ) and acetic acid (8 mℓ) were added into a solution of the crude product prepared in reference example 6 in THF (16 mℓ), and the mixture was stirred for 6 hours under reflux.

The reaction solution was evaporated, and the obtained residue was dissolved in the mixture of ethyl acetate-methylene chloride (4:1; 50 mℓ), and washed with successively, 1N aqueous solution of sodium hydroxide and water. THF (20 mℓ) and methylene chloride (20 mℓ) were added into an organic layer.

This solution was dried, and evaporated to give the crude title compound

TLC: Rf O ($CH_2C\ell_2$:EtOAc = 1:10).

Reference example 7(a) ~ 7(h)

By the same procedure as reference example 7, using each starting material, following compounds having the physical data shown in the Table VIII were given.

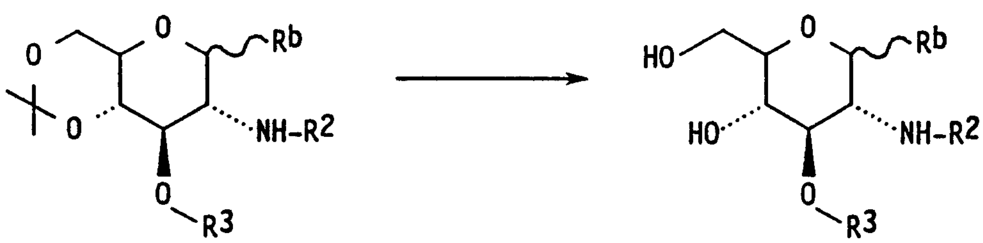

Table VI

| No. | $R^2$, $R^3$, $R^b$ | Name | Starting material | TLC | IR ($cm^{-1}$) |
|---|---|---|---|---|---|
| 7 (a) | $R^2$: $R^3$: $R^b$: 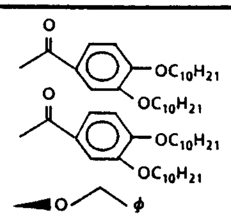 | Benzyl 2-deoxy-2-(3,4-didecyloxybenzoyl)amino-3-O-(3,4-didecyloxybenzoyl)-β-D-glucopyranoside | reference example 5 | | |
| 7 (b) | $R^2$: $R^3$: $R^b$: 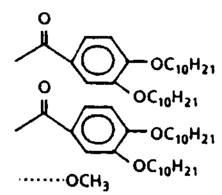 | Methyl 2-deoxy-2-(3,4-didecyloxybenzoyl)amino-3-O-(3,4-didecyloxybenzoyl)-α-D-glucopyranoside | reference example 5 (a) | 0.05 (AcOEt:n-hexane = 1:3) | ν 3400, 3250, 2900, 2820, 1670, 1620, 1590, 1585, 1500, 1450, 1410, 1370, 1320, 1260, 1210, 1120, 1030 |
| 7 (c) | $R^2$: $R^3$: $R^b$: 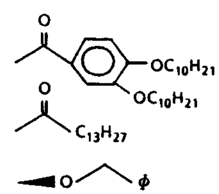 | Benzyl 2-deoxy-2-(3,4-didecyloxybenzoyl)amino-3-O-tetradecanoyl-β-D-glucopyranoside | reference example 6 | | |

Table VI (continued)

| | | | | | |
|---|---|---|---|---|---|
| 7 (d) | R2: R3: Rb: 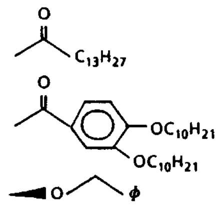 | Benzyl 2-deoxy-2-tetradecanoyl-3-O-(3,4-didecyloxybenzoyl)-β-D-glucopyranoside | reference example 6 (a) | 0.06 (AcOEt: chloroform = 1:10) | ν 3380, 3310, 2900, 2845, 1670, 1645, 1590, 1525, 1460, 1270, 1210, 1090 |
| 7 (e) | R2: R3: Rb: 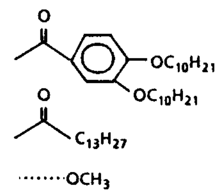 | Methyl 2-deoxy-2-(3,4-didecyloxybenzoyl)amino-3-O-tetradecanoyl-β-D-glucopyranoside | reference example 6 (b) | 0.58 (MeOH:chloroform = 1:9) | ν 3440, 2910, 2840, 1710, 1630, 1490, 1260 |
| 7 (f) | R2: R3: Rb: 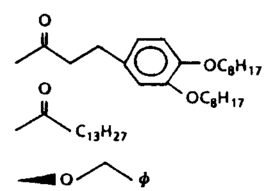 | Benzyl 2-deoxy-2-[3-(3,4-dioctyloxy)phenylpropionyl]amino-3-O-tetradecanoyl-β-D-glucopyranoside | reference example 6 (c) | 0.38 (AcOEt:$CH_2Cl_2$ = 1:2) | ν 3450, 2930, 2855, 1730, 1675, 1505 |

Table VI (continued)

| No. | R1, R2, Rb | Name | Starting material | TLC | IR (cm-1) |
|---|---|---|---|---|---|
| 7 (g) | R2: R3: Rb: 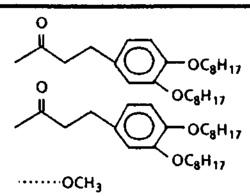 | Methyl 2-deoxy-2-[3-(3,4-dioctyloxy)propanoyl]amino-3-O-[3-(3,4-dioctyloxy)propanoyl]-α-D-glucopyranoside | reference example 6-1 | 0.30 (AcOEt:$CH_2Cl_2$: n-hexane = 3:1:3) | ν 3420, 3370, 2920, 2850, 1720, 1650, 1590, 1510, 1460 |
| 7 (h) | R2: R3: Rb: 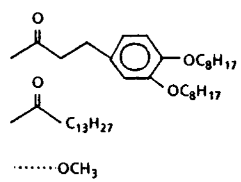 | Methyl 2-deoxy-2-[3-(3,4-dioctyloxy)propanoyl]amino-3-O-tetradecanoyl-α-D-glucopyranoside | reference example 6-1 (a) | 0.10 (AcOEt:$CH_2Cl_2$: n-hexane = 3:1:3) | ν 3350, 3280, 2920, 2860, 1730, 1640 |
| 7 (i) | R2: R3: Rb: 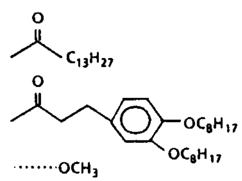 | Methyl 2-deoxy-2-tetradecanoylamino-3-O-[3-(3,4-dioctyloxy)propanoyl]-α-D-glucopyranoside | reference example 6-1 (b) | 0.20 (AcOEt:$CH_2Cl_2$: n-hexane = 3:1:3) | ν 3370, 3300, 2920, 1730, 1640, 1540, 1510 |

Reference example 8

Synthesis of benzyl 2-deoxy-2-(3,4-didecyloxybenzoyl)amino-3-O-tetradecanoyl-6-O-t-butyldimethylsilyl-β-D-glucopyranoside

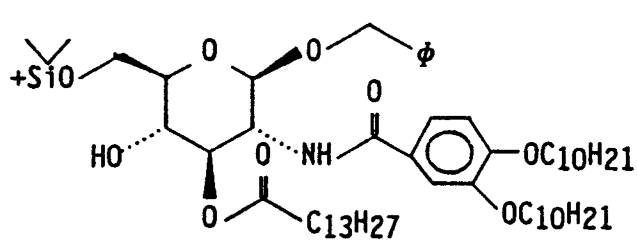

4-(N,N-dimethylamino)pyridine (122 mg) was added to the solution of the compound prepared in reference example 7 in dry pyridine (10 mℓ), the mixture was stirred for 5 hours at room temperature.

The reaction solution was evaporated, and the obtained residue was dissolved in methylene chloride (60 mℓ), and the solution was washed with successively, 1N aqueous solution of hydrochloric acid, water and a saturated aqueous solution of sodium bicarbonate, and then evaporated.

The residue was purified by column chromatography on silica-gel ($CH_2C\ell_2$:EtOAc = 20:1) to give the title compounds (856 mg) having the following physical data.

TLC: Rf 0.66 ($CH_2C\ell_2$:EtOAc = 10:1);

IR ($CHC\ell_3$): $\nu$ 3476, 2940, 2860, 1726, 1660, 1600, 1497, 1462, 1264, 1075, 836 $cm^{-1}$.

Reference example 8(a) ~ 8(e)

By the same procedure as reference example 8, using each starting material, following compounds having the physical data shown in the Table IX were given.

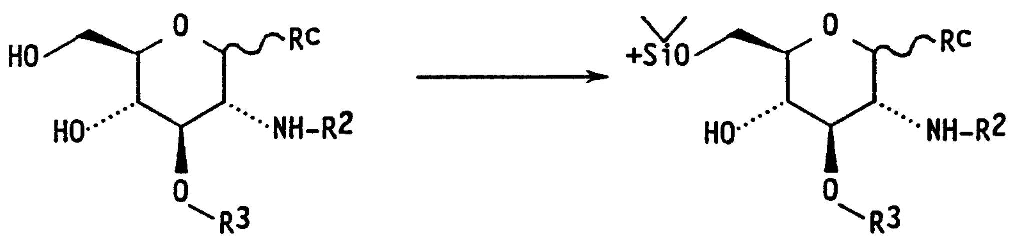

Table IX

| No. | $R^2$, $R^3$, $R^C$ | Name | Starting material | TLC | IR ($cm^{-1}$) |
|---|---|---|---|---|---|
| 8 (a) | $R^2$:<br>$R^3$:<br>$R^C$: 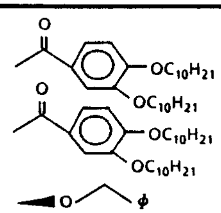 | Benzyl 2-deoxy-2-(3,4-didecyloxybenzoyl)amino-3-O-(3,4-didecyloxybenzoyl)-6-O-t-butyldimethylsilyl-β-D-glucopyranoside | reference example 7 (a) | 0.81 (AcOEt:n-hexane = 3:5) | ν 3400, 3270, 2910, 2850, 1710, 1680, 1630, 1600, 1540, 1510 |
| 8 (b) | $R^2$:<br>$R^3$:<br>$R^C$: 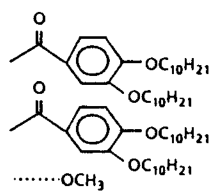 | Methyl 2-deoxy-2-(3,4-didecyloxybenzoyl)amino-3-O-(3,4-didecyloxybenzoyl)-6-O-t-butyldimethylsilyl-α-D-glucopyranoside | reference example 7 (b) | 0.8 (AcOEt:n-hexane = 1:2) | ν 3420, 3300, 2910, 2850, 1670, 1620, 1590, 1570, 1500, 1460, 1420, 1380, 1270, 1210, 1120 1050 |
| 8 (c) | $R^2$:<br>$R^3$:<br>$R^C$: 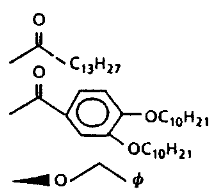 | Benzyl 2-deoxy-2-tetradecanoylamino-3-O-(3,4-didecyloxybenzoyl)-6-O-t-butyldimethylsilyl-β-D-glucopyranoside | reference example 7 (d) | 0.84 (AcOEt:n-hexane = 3:1) | ν 3400, 3275, 2900, 2840, 1675, 1640, 1540, 1460, 1280, 1215, 1115 |

Table IX (continued)

| | | | | | |
|---|---|---|---|---|---|
| 8 (d) | R2:<br>R3:<br>RC: 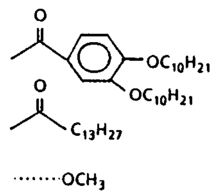 | Methyl 2-deoxy-2-(3,4-didecyloxybenzoyl)amino-3-O-tetradecanoyl-6-O-t-butyldimethylsilyl-α-D-glucopyranoside | reference example 7 (c) | 0.87 (AcOEt:n-hexane = 1:4) | ν 3460, 2910, 2840, 1720, 1645, 1595, 1490, 1460, 1260 |
| 8 (e) | R2:<br>R3:<br>RC: 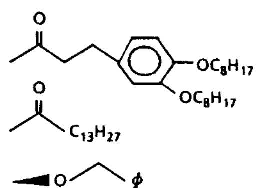 | Benzyl 2-deoxy-2-[3-(3,4-dioctyloxy)phenylpropionyl]amino-3-O-tetradecanoyl-6-O-t-butyldimethylsilyl-β-D-glucopyranoside | reference example 7 (f) | 0.56 (AcOEt:$CH_2Cl_2$ = 1:20) | ν 3450, 2920, 2850, 1720, 1675, 1505, 1460, 1255, 1080, 1060 |

Reference example 9

Synthesis of 2-deoxy-2-[3-(9-phenylnonanoyl)oxytetradecanoyl]amino-3-O-(9-phenylnonanoyl)-1,5-anhydro-D-xylitol

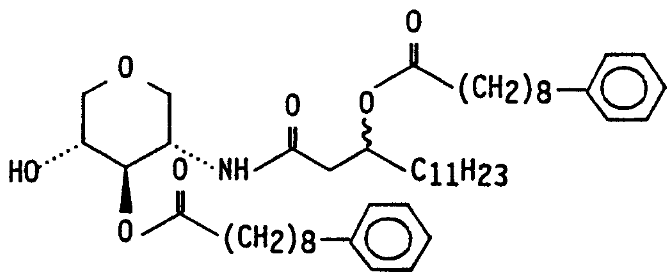

To a stirring solution of the compound (more polar; 260 mg) prepared in reference example 2 dissolved into THF, palladium-carbon (content 10%; 80 mg) was added with small portions. In an atmosphere of hydrogen, the mixture was stirred overnight at 60 - 70 ° C.

After reaction, the reaction solution was filtered. And filtrate was evaporated to give the title compound (more polar).

Reference example 9(a) ~ 9(j)

By the same procedure as reference example, using each starting material, following compounds having the physical data shown in the Table X were given.

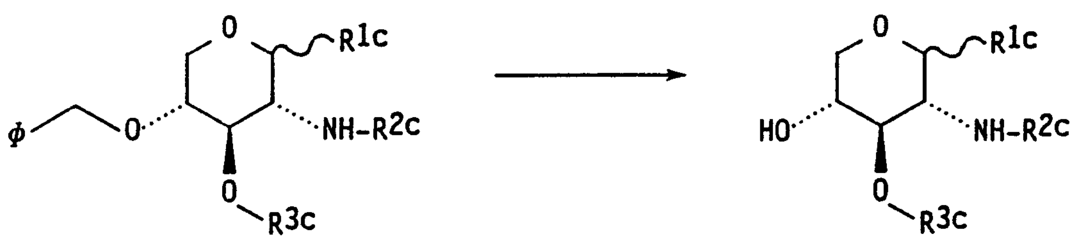

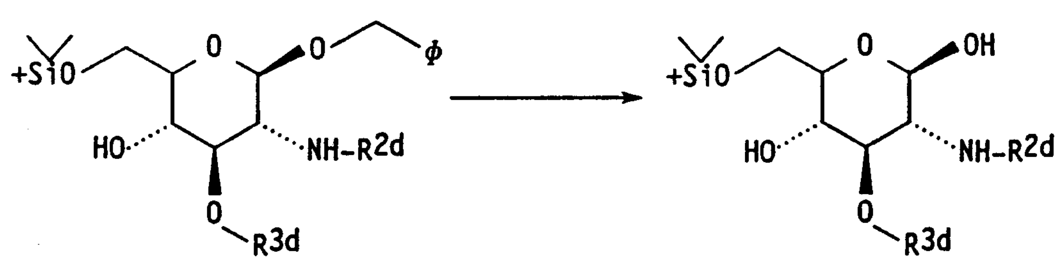

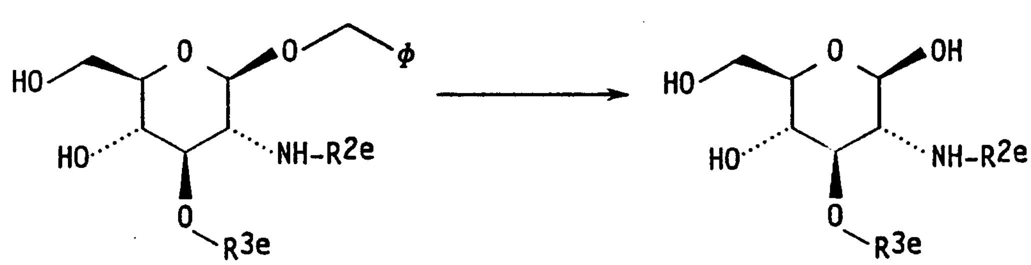

Table X

| No. | R1c,R2c,R3c,R2d,R3d,R2e,R3e | Name | Starting material | TLC | IR (cm-1) |
| --- | --- | --- | --- | --- | --- |
| 9 (a) | R1c: —H<br>R2c: 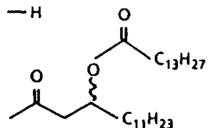<br>R3c: 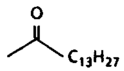 | 2-deoxy-2-(3-tetradecanoyloxytetradecanoyl)amino-3-O-tetradecanoyl-1,5-anhydro-D-xylitol (less polar) | reference example 2 (b) | 0.52 (AcOEt:n-hexane = 3:5) | ν 3450, 3280, 2910, 2850, 1730, 1650, 1530 |
| 9 (b) | R1c: —H<br>R2c: 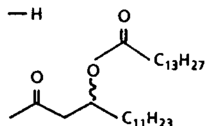<br>R3c: 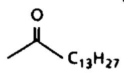 | 2-deoxy-2-(3-tetradecanoyloxytetradecanoyl)amino-3-O-tetradecanoyl-1,5-anhydro-D-xylitol (more polar) | reference example 2 (c) | 0.55 (AcOEt:n-hexane = 1:1) | ν 3450, 3320, 2920, 2850, 1730, 1620, 1550 |
| 9 (c) | R1c: —H<br>R2c: 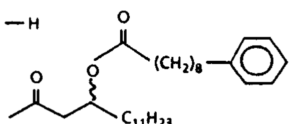<br>R3c: 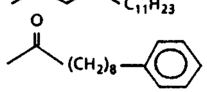 | 2-deoxy-2-[3-(9-phenylnonanoyl)oxytetradecanoyl]amino-3-O-(9-phenylnonanoyl)-1,5-anhydro-D-xylitol (less polar) | reference example 2 (a) | | |

Table X (continued)

| | | | | | |
|---|---|---|---|---|---|
| 9 (d) | R1c: —H<br>R2c: 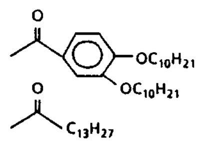<br>R3c: | 2-deoxy-2-(3,4-didecyloxy benzoyl)amino-3-O-tetra decanoyl-1,5-anhydro-D-xylitol | reference example 2 (d) | 0.43 (AcOEt:n-hexane = 1:1) | ν 3430, 3200, 2905, 2840, 1715, 1610, 1540, 1505, 1460, 1360, 1265 |
| 9 (e) | R1c: ·······$OCH_3$<br>R2c: 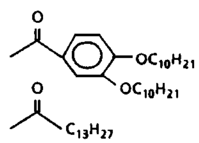<br>R3c: | Methyl 2-deoxy-2-(3,4-didecyloxybenzoyl)amino-3-O-tetradecanoyl-1,5-anhydro-α-D-xyloside | reference example 2 (e) | | |
| 9 (f) | R2e: 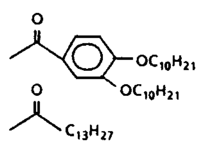<br>R3e: | 2-deoxy-2-(3,4-didecyloxy benzoyl)amino-3-O-tetra decanoyl-D-glucopyranose | reference example 7 (c) | | |
| 9 (g) | R2d: 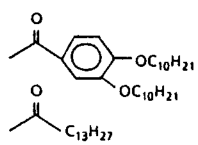<br>R3d: | 2-deoxy-2-(3,4-didecyloxy benzoyl)amino-3-O-tetradecanoyl-6-O-t-butyldimethylsilyl-D-glucopyranose | reference example 8 | 0.50 (AcOEt:n-hexane = 10:1) | |

Table X (continued)

| | | | | | |
|---|---|---|---|---|---|
| 9 (h) | R2d: 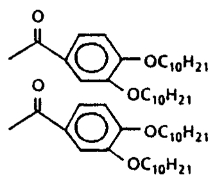 R3d: | 2-deoxy-2-(3,4-didecyloxy benzoyl)amino-3-O-(3,4-didecyloxybenzoyl)-6-O-t-butyldimethylsilyl-D-glucopyranose | reference example 8 (a) | 0.63 (AcOEt:n-hexane = 3:5) | ν 3400, 3270, 2910, 2850, 1710, 1680, 1630, 1600, 1540, 1510 |
| 9 (i) | R2d: 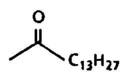 R3d: 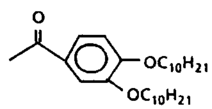 | 2-deoxy-2-tetradecanoylamino-3-O-(3,4-didecyloxybenzoyl)-6-O-t-butyldimethylsilyl-D-glucopyranose | reference example 8 (c) | 0.28 (AcOEt:$CH_2Cl_2$ = 1:10) | ν 3450, 2930, 2820, 1690, 1595, 1500, 1480, 1260 (neat) |
| 9 (j) | R2d: 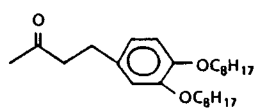 R3d: 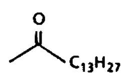 | 2-deoxy-2-[3-(3,4-dioctyloxy)phenylpropyonyl]amino-3-O-tetradecanoyl-6-O-t-butyldimethylsilyl-D-glucopyranose | reference example 8 (e) | 0.40 (AcOEt:$CH_2Cl_2$ = 1:10) | ν 3450, 2965, 2840, 1720, 1660, 1500, 1460, 1250, 1075, 825 |

Example 1

Synthesis of 2-deoxy-2-[3-(9-phenylnonanoyl)oxytetradecanoyl]amino-3-O-(9-phenylnonanoyl)-4-O-sulfo-1,5-anhydro-D-xylitol

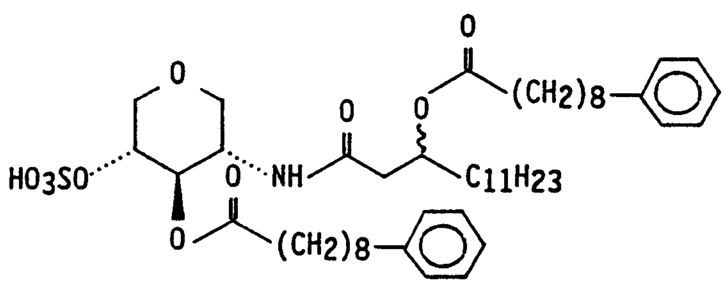

The mixture of a crude products (200 mg) prepared in reference example 9, sulfur trioxide-pyridine complex (144 mg) and pyridine (8 mℓ) was stirred for 3 hours at room temperature.

After reaction, pyridine was separated as the toluene azeotrope. The residue was purified by column chromatography on silica-gel ($CH_2Cℓ_2$:$CH_3OH$ = 10:1) to give the title compounds (more polar; 180 mg) having the following physical data.

TLC: Rf 0.44 ($CH_2Cℓ_2$:$CH_3OH$ = 5:1);

IR : $\nu$ 3450, 3280, 2910, 2850, 1720, 1640, 1520, 1450, 1370, 1250, 1160, 1090, 1060, 990, 810, 740, 690 $cm^{-1}$.

Example 1(a) ~ 1(e)

By the same procedure as example 1, using each starting material, following compounds having the physical data shown in the Table XI were given.

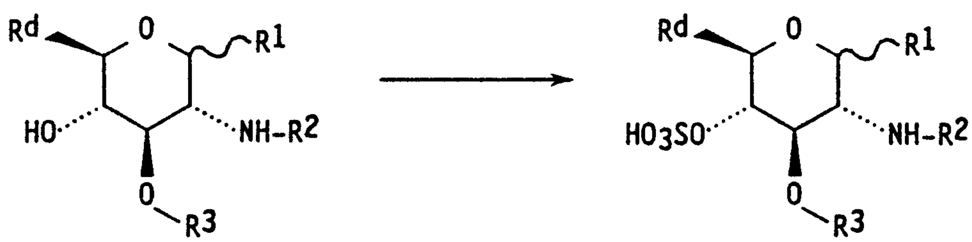

Table XI

| No. | $R^1$, $R^2$, $R^3$, $R^d$ | Name | Starting material | TLC | IR ($cm^{-1}$) |
|---|---|---|---|---|---|
| example 1 (a) | $R^2$: 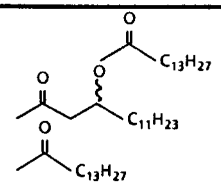 $R^3$: $R^1$,$R^d$: —H | 2-deoxy-2-[3-tetradecanoyl oxytetradecanoyl]amino-3-O-tetradecanoyl-4-O-sulfo-1,5-anhydro-D-xylitol (less polar) | reference example 9 (a) | 0.23 ($CH_2C\ell_2$:MeOH = 10:1) | ν 3450, 2910, 2840, 2670, 1720, 1650, 1530, 1460, 1370, 1260, 1220(S), 1160, 1100, 1050, 990, 800, 710 |
| example 1 (b) | $R^2$: 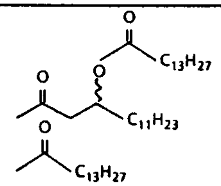 $R^3$: $R^1$,$R^d$: —H | 2-deoxy-2-[3-tetradecanoyl oxytetradecanoyl]amino-3-O-tetradecanoyl-4-O-sulfo-1,5-anhydro-D-xylitol (more polar) | reference example 9 (b) | 0.20 ($CH_2C\ell_2$:MeOH = 10:1) | ν 3450, 3280, 2900, 2840, 1730, 1650, 1540, 1470, 1380, 1250, 1200, 1170, 1100, 1060, 1000, 810 |
| example 1 (c) | $R^2$: 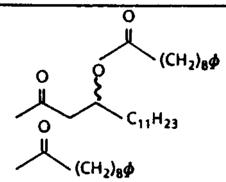 $R^3$: $R^1$,$R^d$: —H | 2-deoxy-2-[3-(9-phenylnonanoyl)oxytetradecanoyl]amino-3-O-(9-phenylnonanoyl)-4-O-sulfo-1,5-anhydro-D-xylitol (less polar) | reference example 9 (c) | 0.49 ($CH_2C\ell_2$:MeOH = 5:1) | ν 3450, 3300, 2910, 2840, 1720, 1650, 1540, 1450, 1380, 1260, 1230, 1170, 1090, 1060, 1000, 810, 740, 690 |

Table XI (continued)

| | | | | | |
|---|---|---|---|---|---|
| example 1 (d) | R2: 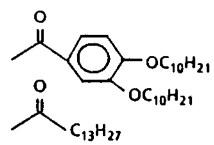 R3: R1,Rd: —H | 2-deoxy-2-(3,4-didecyloxybenzoyl)amino-3-O-tetradecanoyl-4-O-sulfo-1,5-anhydro-D-xylitol | reference example 9 (d) | 0.33 (chloroform:MeOH = 10:1) | ν 3425, 3360, 2910, 1725, 1635, 1505, 1260, 1215 |
| example 1 (e) | R1: ·······$OCH_3$ R2: 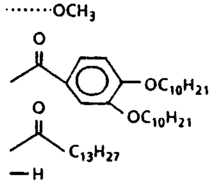 R3: Rd: —H | Methyl 2-deoxy-2-(3,4-didecyloxybenzoyl)amino-3-O-tetradecanoyl-4-O-sulfo-1,5-anhydro-α-D-xyloside | reference example 9 (e) | 0.30 ($CH_2Cl_2$:MeOH = 10:1) | ν 3420, 3350, 2910, 1720, 1640, 1510, 1270, 1210 |

Table XI (continued)

| | | | | | |
|---|---|---|---|---|---|
| reference example 10 (a) | R1: R2: R3: Rd: 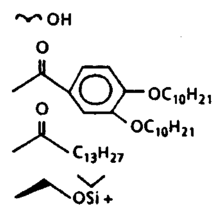 | 2-deoxy-2-(3,4-didecyloxybenzoyl)amino-3-O-tetradecanoyl-4-O-sulfo-6-O-t-butyldimethylsilyl-D-glucopyranose | reference example 9 (g) | 0.20 ($CH_2C\ell_2$:MeOH = 10:1) | |
| reference example 10 (b) | R1: R2: R3: Rd: 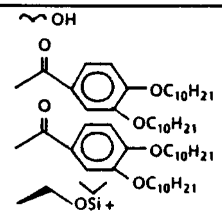 | 2-deoxy-2-(3,4-didecyloxy benzoyl)amino-3-O-(3,4-didecyloxybenzoyl)-4-O-sulfo-6-O-t-butyldimethyl silyl-D-glucopyranose | reference example 9 (h) | | |

Table XI (continued)

| | | | | | |
|---|---|---|---|---|---|
| reference example 10 (c) | R1:<br>R2:<br>R3:<br>Rd: 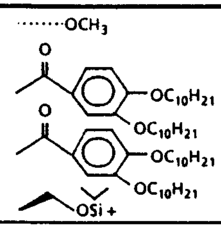 | Methyl 2-deoxy-2-(3,4-didecyloxybenzoyl)amino-3-O-(3,4-didecyloxybenzoyl)-4-O-sulfo-6-O-t-butyldimethylsilyl-D-glucopyranoside | reference example 8 (b) | 0.68 ($CH_2Cl_2$:MeOH = 20:3) | |
| reference example 10 (d) | R1:<br>R2:<br>R3:<br>Rd: 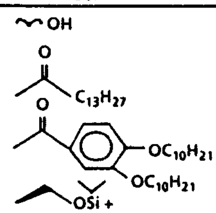 | 2-deoxy-2-(tetradecanoyl amino)-3-O-(3,4-didecyloxy benzoyl)-4-O-sulfo-6-O-t-butyldimethylsilyl-D-glucopyranose | reference example 9 (i) | | |
| reference example 10 (e) | R1:<br>R2:<br>R3:<br>Rd: 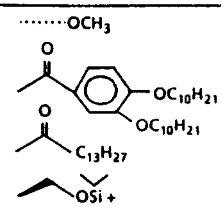 | Methyl 2-deoxy-2-(3,4-didecyloxybenzoyl)amino-3-O-tetradecanoyl-4-O-sulfo-6-O-t-butyldimethylsilyl-D-glucopyranoside | reference example 8 (d) | | |

Table XI (continued)

| | | | | | |
|---|---|---|---|---|---|
| reference example 10 (f) | R1:<br>R2:<br>R3:<br>Rd: 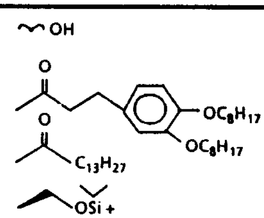 | 2-deoxy-2-[3-(3,4-dioctyloxy)phenylpropionyl]amino-3-O-tetradecanoyl-4-O-sulfo-6-O-t-butyldimethylsilyl-D-glucopyranose | reference example 9 (j) | 0.5 ($CH_2Cl_2$:MeOH = 10:1) | |

Example 2

Synthesis of 2-deoxy-2-(3,4-didecyloxybenzoyl)amino-3-O-tetradecanoyl-4-O-sulfo-D-glucopyranose

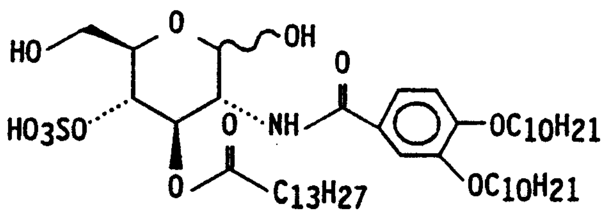

The crude products prepared in reference example 10(a) was dissolved in a mixed solvent of acetic acid (10 mℓ), THF (10 mℓ) and water (5 mℓ), and stirred for 30 minutes at 50 °C. After reaction, the reaction solution was evaporated, and acetic acid was separated as the toluene azeotrope.

And further the reaction solution was evaporated. The residue was purified by column chromatography on silica-gel ($CH_2Cℓ_2$:$CH_3OH$ = 20:3). To the compound obtained, dry dioxan was added, and the solution was freeze-dried to give the title compound (261 mg) having the following physical data.

TLC: Rf 0.15 ($CH_2Cℓ_2$:$CH_3OH$ = 20:3);

IR : ν 3450, 2940, 2860, 1730, 1632, 1601, 1580, 1522, 1507, 1460, 1272, 1227, 1130, 1043, 996, 816, 765, 720, 600 $cm^{-1}$.

Example 2(a) ~ 2(e)

By the same procedure as reference example 2, using each starting material, following compounds having the physical data shown in the Table XII were given.

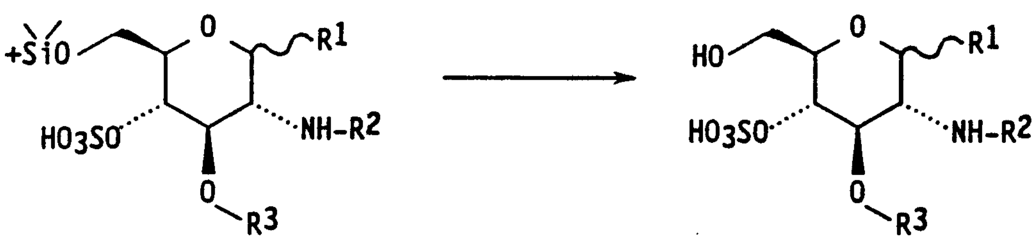

Table XII

| No. | $R^1$, $R^2$, $R^3$ | Name | Starting material | TLC | IR ($cm^{-1}$) |
|---|---|---|---|---|---|
| 2 (a) | $R^1$: $R^2$: $R^3$: 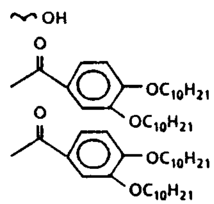 | 2-deoxy-2-(3,4-didecyloxybenzoyl)amino-3-O-(3,4-didecyloxybenzoyl)-4-O-sulfo-D-glucopyranose | reference example 10 (b) | 0.10 ($CH_2Cl_2$:MeOH = 9:1) | ν 3420, 2910, 2850, 1700, 1630, 1600, 1510, 1460, 1420, 1380, 1270, 1210, 1130, 1030, 990 |
| 2 (b) | $R^1$: $R^2$: $R^3$: 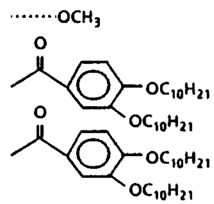 | Methyl 2-deoxy-2-(3,4-didecyloxybenzoyl)amino-3-O-(3,4-didecyloxybenzoyl)-4-O-sulfo-α-D-glucopyranoside | reference example 10 (c) | 0.50 ($CH_2Cl_2$:MeOH = 20:3) | ν 3400, 2920, 2850, 1690, 1630, 1600, 1500, 1460, 1420, 1370, 1260, 1210, 1120, 1030, 990 |
| 2 (c) | $R^1$: $R^2$: $R^3$: 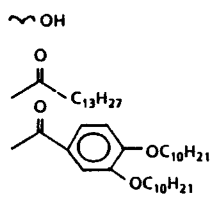 | 2-deoxy-2-tetradecanoyl amino-3-O-(3,4-didecyloxy benzoyl)amino-4-O-sulfo-D-glucopyranose | reference example 10 (d) | 0.29 ($CH_2Cl_2$:MeOH = 20:3) | ν 3350, 2910, 2835, 1710, 1605, 1265, 1205 |

Table XII (continued)

| | | | | | |
|---|---|---|---|---|---|
| 2 (d) | R1: R2: R3: 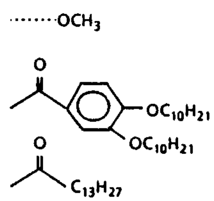 | Methyl 2-deoxy-2-(3,4-didecyloxybenzoyl)amino-3-O-tetradecanoyl-4-O-sulfo-α-D-glucopyranoside | reference example 10 (e) | 0.24 ($CH_2C\ell_2$:MeOH = 20:3) | ν 3425, 2910, 2840, 1720, 1635, 1495, 1260 |
| 2 (e) | R1: R2: R3: 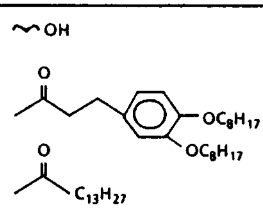 | 2-deoxy-2-[3-(3,4-dioctyloxy)phenylpropionyl]amino-3-O-tetradecanoyl-4-O-sulfo-D-glucopyranose | reference example 10 (f) | 0.29 ($CH_2C\ell_2$:MeOH = 20:3) | ν 3400, 2915, 2850, 1720, 1640, 1520, 1460, 1260 |

Example 3

Synthesis of 2-deoxy-2-(3,4-didecyloxybenzoyl)amino-3-O-tetradecanoyl-4,6-O-disulfo-D-glucopyranose

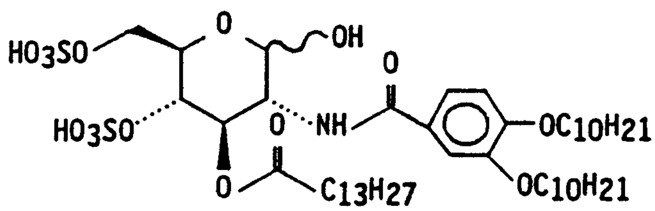

By the same procedure as example 1, using the compound (0.57 g) prepared in reference example 9(f), the title compound having the following physical data was given. In this case, two-fold amount of a sulfur trioxide-pyridine complex which was used in example 1 was used.

TLC: Rf 0.16 ($CH_2C\ell_2$:MeOH = 4:1);

IR : $\nu$ 3400, 2920, 2850, 1730, 1630, 1600, 1570, 1540, 1510, 1460, 1380, 1340, 1260, 1210, 1000 $cm^{-1}$

Example 3(a) ~ 3(d)

By the same procedure as example 3, using each starting material, following compounds having the physical data shown in the Table XIII were given.

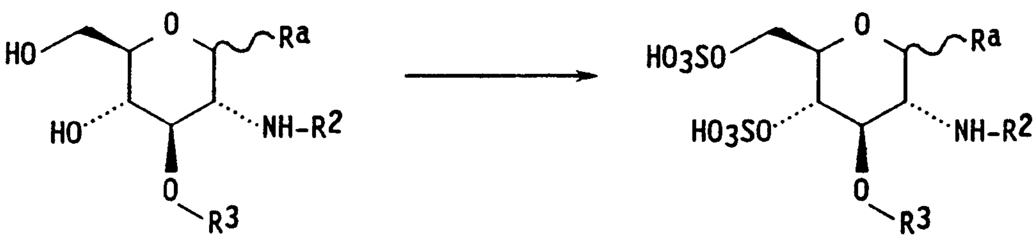

Table XIII

| No. | $R^1$, $R^2$, $R^3$ | Name | Starting material | TLC | IR ($cm^{-1}$) |
|---|---|---|---|---|---|
| 3 (a) | R1: 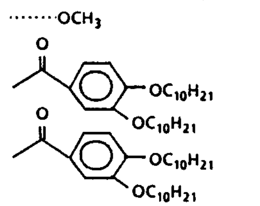 R2: R3: | Methyl 2-deoxy-2-(3,4-didecyloxybenzoyl)amino-3-O-(3,4-didecyloxybenzoyl)-4,6-O-disulfo-α-D-glucopyranoside | reference example 7 (b) | 0.50 ($CH_2Cl_2$:MeOH = 4:1) | ν 3400, 2930, 2850, 1690, 1630, 1600, 1510, 1460, 1430, 1270, 1210, 1130, 1010, 870, 820, 760, 600 |
| 3 (b) | R1: 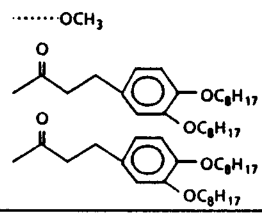 R2: R3: | Methyl 2-deoxy-2-[3-(3,4-dioctyloxyphenyl)propanoyl]amino-3-O-[3-(3,4-dioctyloxyphenyl)propanoyl]-4,6-O-disulfo-α-D-glucopyranoside | reference example 7 (g) | 0.15 ($CH_2Cl_2$:MeOH = 20:3) | ν 3410, 2920, 2850, 1720, 1630, 1510, 1460, 1240, 1010 |
| 3 (c) | R1: 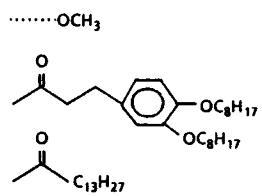 R2: R3: | Methyl 2-deoxy-2-[3-(3,4-dioctyloxyphenyl)propanoyl]amino-3-O-tetradecanoyl-4,6-O-disulfo-α-D-glucopyranoside | reference example 7 (h) | 0.14 ($CH_2Cl_2$:MeOH = 20:3) | ν 3400, 2920, 2850, 1720, 1630, 1510, 1460, 1220, 1000, 920 |

Table XIII (continued)

| | | | | | |
|---|---|---|---|---|---|
| 3 (d) | R1: 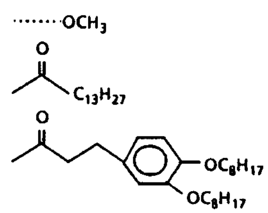 R2: R3: | Methyl 2-deoxy-2-tetradecanoylamino-3-O-[3-(3,4-dioctyloxyphenyl) propanoyl]-4,5-O-disulfo-α-D-glucopyranoside | reference example 7 (i) | 0.16 ($CH_2Cl_2$:MeOH = 20:3) | ν 3400, 2910, 2840, 1720, 1630, 1500, 1460, 1370, 1220, 1050 |

## Claims

**Claims for the following Contracting States : AT, BE, CH, DE, FR, GB, IT, LI, LU, NL, SE**

**1.** A glucopyranose derivative of general formula:

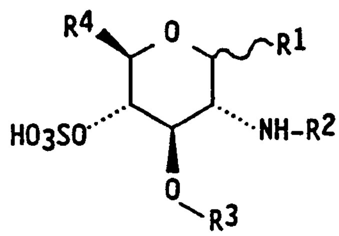

(I)

wherein $R^1$ represents a hydrogen atom, a hydroxy group or an alkoxy group of from 1 to 4 carbon atom(s),
$R^2$ represents the group represented by A, B, D or E, wherein
A represents a general formula:

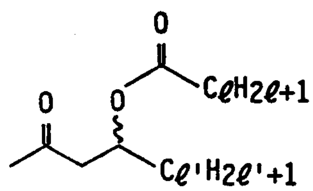

,

B represents a general formula:

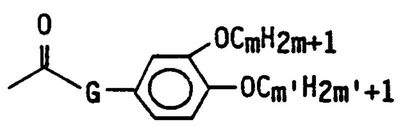

,

D represents a general formula:

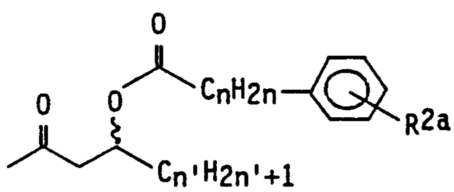

and
E represents a general formula:

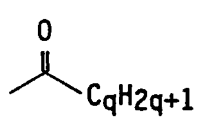

;

in each formula, $\ell$ and q each represents an integer of 11~15, m and m' each represents an integer of 6~12, n represents an integer of 6~10, $\ell$' and n' each represents an integer of 9~13,
G represents a single bond or an alkylene group of from 1 to 4 carbon atom(s),

$R^{2a}$ represents a hydrogen atom, an alkyl group or alkoxy group of from 1 to 7 carbon atom(s) or a halogen atom;
$R^3$ represents the group represented by L, M or Q, wherein
L represents a general formula:

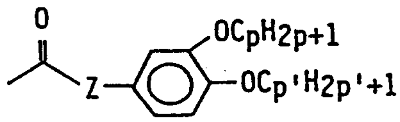

,

M represents a general formula:

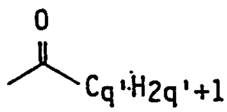

and
Q represents a general formula:

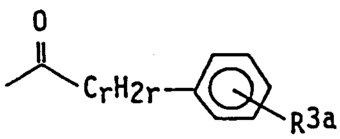

;

in each formula, Z represents a single bond or an alkylene group of from 1 to 4 carbon atom(s), p and p' each represents an integer of 6~12,
q' represents an integer of 11~15, r represents an integer of 6~10,
$R^{3a}$ represents a hydrogen atom, an alkyl group or alkoxy group of from 1 to 7 carbon atom(s) or a halogen atom;
$R^4$ represents a hydrogen atom, a hydroxymethyl group or sulfoxymethyl ($-CH_2OSO_3H$) group;
with the proviso that, when $R^4$ represents a hydroxymethyl group,
(i) (A,M), (A,Q), (D,M), (D,Q), (E,M) and (E,Q) as the combination of ($R^2$, $R^3$) are excluded, (ii) m and m' in the formula (B) of $R^2$ represent an integer of 8 to 12 and (iii) p and p' in the formula (L) of $R^3$ represent an integer of 8 to 12,
and when $R^4$ represents a sulfoxymethyl group, (A,M) and (E,M) as the combination of ($R^2$, $R^3$) are excluded,
or non-toxic salts thereof.

**2.** A compound according to claim 1, wherein $R^4$ is a hydrogen atom.

**3.** A compound according to claim 1 or claim 2, wherein $R^2$ is the general formula represented by B.

**4.** A compound according to claim 1 or claim 3, wherein $R^3$ is the general formula represented by L.

**5.** A compound according to claims 1 to 4, which is selected from the group consisting of
2-deoxy-2-(3-tetradecanoyloxytetradecanoyl)amino-3-O-tetradecanoyl-4-O-sulfo-1,5-anhydro-D-xylitol,
2-deoxy-2-[3-(9-phenylnonanoyl)oxytetradecanoyl]amino-3-O-(9-phenylnonanoyl)-4-O-sulfo-1,5-anhydro-D-xylitol,
2-deoxy-2-(3,4-didecyloxybenzoyl)amino-3-O-tetradecanoyl-4-O-sulfo-1,5-anhydro-D-xylitol,
Methyl 2-deoxy-2-(3,4-didecyloxybenzoyl)amino-3-O-tetradecanoyl-4-O-sulfo-5-anhydro-D-glucopyranoside,
2-deoxy-2-(3,4-didecyloxybenzoyl)amino-3-O-tetradecanoyl-4-O-sulfo-D-glucopyranose,
2-deoxy-2-(3,4-didecyloxybenzoyl)amino-3-O-(3,4-didecyloxybenzoyl)-4-O-sulfo-D-glucopyranose,
Methyl 2-deoxy-2-(3,4-didecyloxybenzoyl)amino-3-O-(3,4-didecyloxybenzoyl)-4,6-disulfo-α-D-glucopyranoside,

Methyl 2-deoxy-2-(3,4-didecyloxybenzoyl)amino-3-O-(3,4-didecyloxybenzoyl)-4-O-sulfo-α-D-glucopyranoside,
2-deoxy-2-(3,4-didecyloxybenzoyl)amino-3-O-tetradecanoyl-4,6-O-disulfo-D-glucopyranoside,
Methyl 2-deoxy-2-(3,4-didecyloxybenzoyl)amino-3-O-tetradecanoyl-4-O-sulfo-D-glucopyranoside,
2-deoxy-2-tetradecanoylamino-3-O-(3,4-didecyloxybenzoyl)-4-O-sulfo-D-glucopyranose,
2-deoxy-2-[3-(3,4-dioctyloxy)phenylpropionyl]amino-3-O-tetradecanoyl-4-O-sulfo-D-glucopyranose,
Methyl 2-deoxy-2[3-(3,4-dioctyloxyphenyl)propanoyl]amino-3-O-[3-(3,4-dioctyloxyphenyl)propanoyl]-4,6-disulfo-α-D-glucopyranoside,
Methyl 2-deoxy-2-[3-(3,4-dioctyloxyphenyl)propanoyl]amino-3-O-tetradecanoyl-4,6-O-disulfo-α-D-glucopyranoside and
Methyl 2-deoxy-2-tetradecanoylamino-3-O-[3-(3,4-dioctyloxyphenyl)propanoyl]-4,5-O-disulfo-α-D-glucopyranoside.

**6.** A process for the preparation of the compound of the general formula:

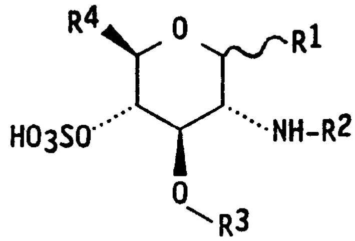

(I)

wherein $R^1$ represents a hydrogen atom, a hydroxy group or an alkoxy group of from 1 to 4 carbon atom(s),
$R^2$ represents the group represented by A, B, D or E
A represents a general formula:

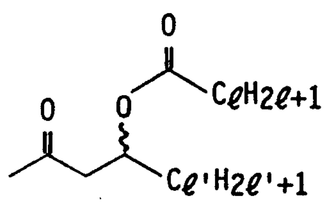

,

B represents a general formula:

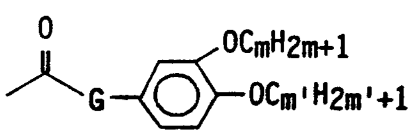

,

D represents a general formula:

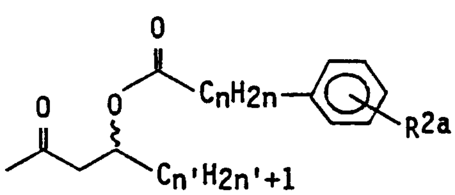

and
E represents a general formula:

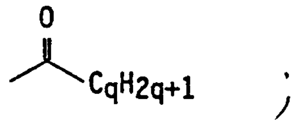

in each formula, ℓ and q each represents an integer of 11~15, m and m' each represents an integer of 6~12, n represents an integer of 6~10, ℓ' and n' each represents an integer of 9~13,
G represents a single bond or an alkylene group of from 1 to 4 carbon atom(s),
$R^{2a}$ represents a hydrogen atom, an alkyl group or alkoxy group of from 1 to 7 carbon atom(s) or a halogen atom;
$R^3$ represents the group represented by L, M or Q, wherein
L represents a general formula:

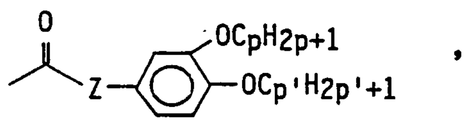

M represents a general formula:

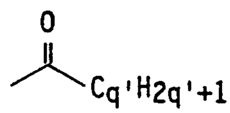

and
Q represents a general formula:

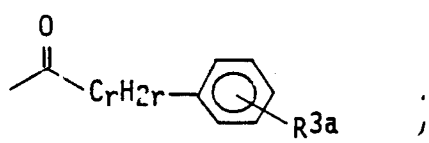

in each formula, Z represents a single bond or an alkylene group of from 1 to 4 carbon atom(s), p and p' each represents an integer of 6~12,
q' represents an integer of 11~15, r represents an integer of 6~10,
$R^{3a}$ represents a hydrogen atom, an alkyl group or alkoxy group of from 1 to 7 carbon atom(s) or a halogen atom;
$R^4$ represents a hydrogen atom, a hydroxymethyl group or a sulfoxymethyl group,
with the proviso that, when $R^4$ represents a hydroxymethyl group,
(i) (A,M), (A,Q), (D,M), (D,Q), (E,M) and (E,Q) as the combination of ($R^2$, $R^3$) are excluded, (ii) m and m' in the formula (B) of $R^2$ represent an integer of 8 to 12 and (iii) p and p' in the formula (L) of $R^3$ represent an integer of 8 to 12,

and when $R^4$ represents a sulfoxymethyl group, (A,M) and (E,M) as the combination of ($R^2$, $R^3$) are excluded,
which is characterized:
by introducing a sulfo group into the hydroxy group at 4-position of a compound of the general formula:

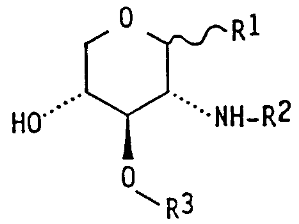

(II)

wherein all of the symbols are the same meaning as defined hereinbefore, by introducing sulfo groups into the hydroxy groups at 4- and 6-positions of a compound of the general formula:

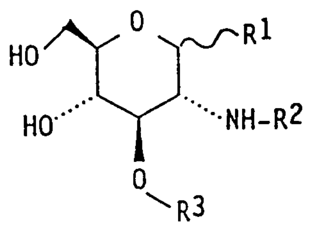

(III)

wherein all of the symbols are the same meaning as defined hereinbefore, by hydrolyzing a compound of the general formula:

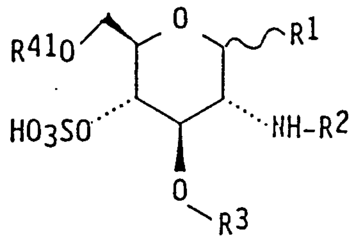

(IV)

wherein $R^{41}$ represents a trialkylsilyl group, and the other symbols have the same meanings as defined hereinbefore, in acidic conditions.

**7.** A pharmaceutical composition for use in the enhancement of immunity characterized by comprising as active ingredient, a compound of the general formula (I) depicted in claim 1 together with a pharmaceutical carrier and/or coating.

**8.** A pharmaceutical composition for use in the treatment of tumor characterized by comprising as active ingredient, a compound of the general formula (I) depicted in claim 1 together with a pharmaceutical carrier and/or coating.

**9.** A compound of the general formula (I) depicted in claim 1 for use in the manufacture of medicament for the enhancement of immunity.

**10.** A compound of the general formula (I) depicted in claim 1 for use in the manufacture of medicament for the treatment of tumor.

**Claims for the following Contracting States : ES, GR**

1. A process for the preparation of the compound of the general formula:

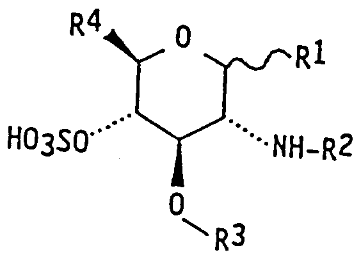

(I)

wherein $R^1$ represents a hydrogen atom, a hydroxy group or an alkoxy group of from 1 to 4 carbon atom(s),
$R^2$ represents the group represented by A, B, D or E, wherein
A represents a general formula:

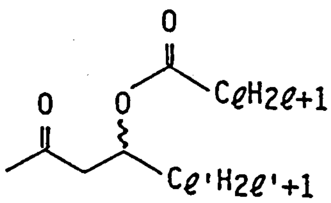

,

B represents a general formula:

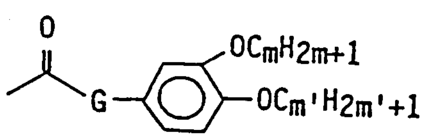

,

D represents a general formula:

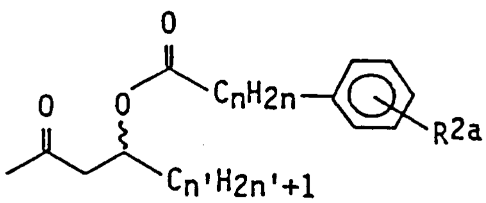

and
E represents a general formula:

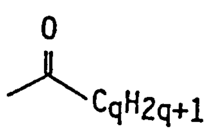

in each formula, l and q each represents an integer of 11-15, m and m' each represents an integer of 6-12, n represents an integer of 6-10, l' and n' each represents an integer of 9-13,

G represents a single bond or an alkylene group of from 1 to 4 carbon atom(s), $R^{2a}$ represents a hydrogen atom, an alkyl group or alkoxy group of from 1 to 7 carbon atom(s) or a halogen atom;
$R^3$ represents the group represented by L, M or Q, wherein
L represents a general formula:

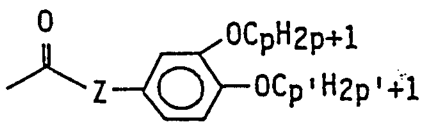

M represents a general formula:

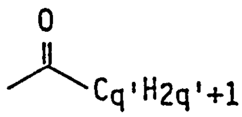

and
Q represents a general formula:

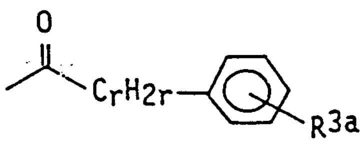

in each formula, Z represents a single bond or an alkylene group of from 1 to 4 carbon atom(s), p and p' each represents an integer of 6-12, q' represents an integer of 11-15, r represents an integer of 6-10, $R^{3a}$ represents a hydrogen atom, an alkyl group or a alkoxy group of from 1 to 7 carbon atom(s) or a halogen atom;
$R^4$ represents a hydrogen atom, a hydroxymethyl group or a sulfoxymethyl group,
with the proviso that, when $R^4$ represents a hydroxymethyl group,

(i) (A,M), (A,Q), (D,M), (D,Q), (E,M) and (E,Q) as the combination of ($R^2$ $R^3$) are excluded,
(ii) m and m' in the formula (B) of $R^2$ represent an integer of 8 to 12 and
(iii) p and p' in the formula (L) of $R^3$ represent an integer of 8 to 12,

and when $R^4$ represents a sulfoxymethyl group, (A,M) and (E,M) as the combination of ($R^2$, $R^3$) are excluded, which is characterized:
by introducing a sulfo group into the hydroxy group at 4-position of a compound of the general formula:

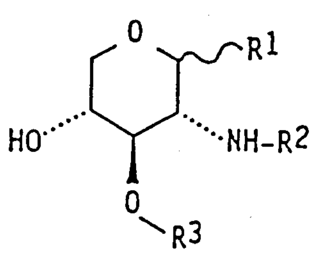

(II)

wherein all of the symbols are the same meaning as defined hereinbefore,
by introducing sulfo groups into the hydroxy groups at 4- and 6-positions of a compound of the general formula:

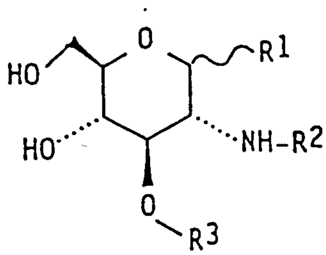

(III)

wherein all of the symbols are the same meaning as defined hereinbefore,
by hydrolyzing a compound of the general formula:

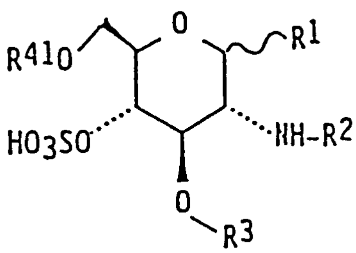

(IV)

wherein $R^{41}$ represents a trialkylsilyl group, and the other symbols have the same meanings as defined hereinbefore,
in acidic conditions.

2. A process for the preparation of the compound according to claim 1, wherein $R^4$ is a hydrogen atom.

3. A process for the preparation of the compound according to claim 1 or claim 2, wherein $R^2$ is the general formula represented by B.

4. A process for the preparation of the compound according to claim 1 or claim 3, wherein $R^3$ is the general formula represented by L.

5. A process for the preparation of the compound according to claims 1 to 4, which is selected from the group consisting of
2-deoxy-2-(3-tetradecanoyloxytetradecanoyl)amino-3-O-tetradecanoyl-4-O-sulfo-1,5-anhydro-D-xylitol,
2-deoxy-2-[3-(9-phenylnonanoyl)oxytetradecanoyl]amino-3-O-(9-phenylnonanoyl)-4-O-sulfo-1,5-anhydro-D-xylitol,
2-deoxy-2-(3,4-didecyloxybenzoyl)amino-3-O-tetradecanoyl-4-O-sulfo-1,5-anhydro-D-xylitol,
Methyl 2-deoxy-2-(3,4-didecyloxybenzoyl)amino-3-O-tetradecanoyl-4-O-sulfo-5-anhydro-D-glucopyranoside,
2-deoxy-2-(3,4-didecyloxybenzoyl)amino-3-O-tetradecanoyl-4-O-sulfo-D-glucopyranose,
2-deoxy-2-(3,4-didecyloxybenzoyl)amino-3-O-(3,4-didecyloxybenzoyl)-4-O-sulfo-D-glucopyranose,
Methyl 2-deoxy-2-(3,4-didecyloxybenzoyl)amino-3-O-(3,4-didecyloxybenzoyl)-4,6-disulfo-α-D-glucopyranoside,
Methyl 2-deoxy-2-(3,4-didecyloxybenzoyl)amino-3-O-(3,4-didecyloxybenzoyl)-4-O-sulfo-α-D-glucopyranoside,
2-deoxy-2-(3,4-didecyloxybenzoyl)amino-3-O-tetradecanoyl-4,6-O-disulfo-D-glucopyranoside,
Methyl 2-deoxy-2-(3,4-didecyloxybenzoyl)amino-3-O-tetradecanoyl-4-O-sulfo-D-glucopyranoside,
2-deoxy-2-tetradecanoylamino-3-O-(3,4-didecyloxybenzoyl)-4-O-sulfo-D-glucopyranose,
2-deoxy-2-[3-(3,4-dioctyloxy)phenylpropionyl]amino-3-O-tetradecanoyl-4-O-sulfo-D-glucopyranose,
Methyl 2-deoxy-2-[3-(3,4-dioctyloxyphenyl)propanoyl]-amino-3-O-[3-(3,4-dioctyloxyphenyl)-propanoyl]-4,6-disulfo-α-D-glucopyranoside,
Methyl 2-deoxy-2-[3-(3,4-dioctyloxyphenyl)-propanoyl]-amino-3-O-tetradecanoyl-4,6-O-disulfo-α-D-glucopyranoside and
Methyl 2-deoxy-2-tetradecanoylamino-3-O-[3-(3,4-dioctyloxyphenyl)propanoyl]-4,5-O-disulfo-α-D-glucopyranoside.

6. A process for the preparation of the pharmaceutical composition for use in the enhancement of immunity characterized by comprising as active ingredient, a compound of the general formula (I) depicted in claim 1 together with a pharmaceutical carrier and/or coating.

7. A process for the preparation of the pharmaceutical composition for use in the treatment of tumor characterized by comprising as active ingredient, a compound of the general formula (I) depicted in claim 1 together with a pharmaceutical carrier and/or coating.

**Patentansprüche**
**Patentansprüche für folgende Vertragsstaaten : AT, BE, CH, DE, FR, GB, IT, LI, LU, NL, SE**

1. Glucopyranosederivat der allgemeinen Formel:

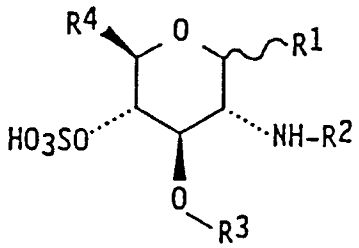

(I)

worin bedeuten:
$R^1$ ein Wasserstoffatom, eine Hydroxygruppe oder eine Alkoxygruppe mit 1 bis 4 Kohlenstoffatom(en),
$R^2$ die Gruppe A, B, D oder E mit
A gleich einer allgemeinen Formel:

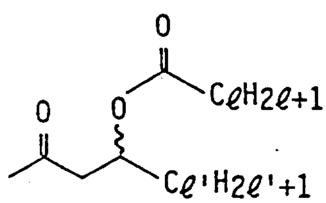

,

B gleich einer allgemeinen Formel:

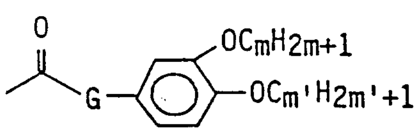

,

D gleich einer allgemeinen Formel:

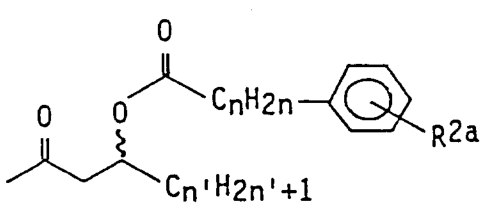

und
E gleich einer allgemeinen Formel:

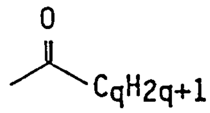 ,

wobei in jeder Formel l und q jeweils ganze Zahlen von 11 - 15, m und m' jeweils ganze Zahlen von 6 - 12, n eine ganze Zahl von 6 - 10 und l' und n' jeweils ganze Zahlen von 9 - 13 bedeuten,
G eine Einfachbindung oder eine Alkylengruppe mit 1 - 4 Kohlenstoffatom(en) darstellt und
$R^{2a}$ ein Wasserstoffatom, eine Alkylgruppe oder eine Alkoxygruppe mit 1 bis 7 Kohlenstoffatom(en) oder ein Halogenatom bedeutet,
$R^3$ die Gruppe L, M oder Q mit
L gleich einer allgemeinen Formel:

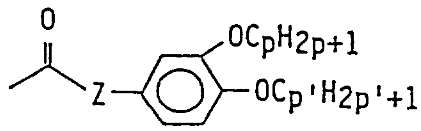 ,

M gleich einer allgemeinen Formel:

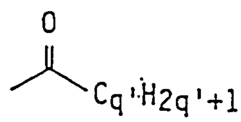

und
Q gleich einer allgemeinen Formel:

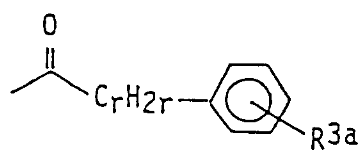 , ;

wobei in jeder Formel Z eine Einfachbindung oder eine Alkylengruppe mit 1 bis 4 Kohlenstoffatom(en) bedeutet,
p und p' jeweils ganze Zahlen von 6 - 12, q' eine ganze Zahl von 11 - 15 und r eine ganze Zahl von 6 - 10 bedeuten, und
$R^{3a}$ für ein Wasserstoffatom, eine Alkylgruppe oder eine Alkoxygruppe mit 1 bis 7 Kohlenstoffatom(en) oder ein Halogenatom steht,
$R^4$ ein Wasserstoffatom, eine Hydroxymethylgruppe oder Sulfoxymethyl ($-CH_2OSO_3H$)-Gruppe,
wobei gilt, daß wenn $R^4$ für eine Hydroxymethylgruppe steht,

(i) (A, M), (A,Q), (D,M), (D,Q), (E,M) und (E,Q) als Kombination von ($R^2$, $R^3$) ausgeschlossen sind,
(ii) m und m' in der Formel (B) von $R^2$ für eine ganze Zahl von 8 - 12 stehen und
(iii) p und p' in der Formel (L) von $R^3$ eine ganze Zahl von 8 - 12 bedeuten, und

wenn $R^4$ für eine Sulfoxymethylgruppe steht, (A,M) und (E,M) als Kombination von ($R^2$ $R^3$) ausgeschlossen sind, oder nicht-toxische Salze davon.

**2.** Verbindung nach Anspruch 1, wobei $R^4$ für ein Wasserstoffatom steht.

**3.** Verbindung nach Anspruch 1 oder Anspruch 2, wobei $R^2$ in der allgemeinen Formel durch B dargestellt ist.

4. Verbindung nach Anspruch 1 oder Anspruch 3, wobei $R^3$ in der allgemeinen Formel durch L dargestellt ist.

5. Verbindung nach Ansprüchen 1 bis 4, ausgewählt aus der Gruppe
2-Desoxy-2-(3-tetradecanoyloxytetradecanoyl)-amino-3-0-tetradecanoyl-4-0-sulfo-1,5-anhydro-D-xylit,
2-Desoxy-2-[3-(9-phenylnonanoyl)-oxytetradecanoyl]-amino-3-0-(9-phenylnonanoyl)-4-0-sulfo-1,5-anhydro-D-xylit,
2-Desoxy-2-(3,4-didecyloxybenzoyl)-amino-3-0-tetradecanoyl-4-0-sulfo-1,5-anhydro-D-xylit,
Methyl-2-desoxy-(3,4-didecyloxybenzoyl)-amino-3-0-tetradecanoyl-4-0-sulfo-5-anhydro-D-glucopyranosid,
2-Desoxy-2-(3,4-didecyloxybenzoyl)-amino-3-0-tetradecanoyl-4-O-sulfo-D-glucopyranose,
2-Desoxy-2-(3,4-didecyloxybenzoyl)-amino-3-O-(3,4-didecyloxybenzoyl)-4-O-sulfo-D-glucopyranose,
Methyl-2-desoxy-2-(3,4-didecyloxybenzoyl)-amino-3-O-(3,4-didecyloxybenzoyl)-4,6-disulfo-α-D-glucopyranosid,
Methyl-2-desoxy-2-(3,4-didecyloxybenzoyl)-amino-3-O-(3,4-didecyloxybenzoyl)-4-0-sulfo-α-D-glucopyranosid,
2-Desoxy-2-(3,4-didecyloxybenzoyl)-amino-3-0-tetradecanoyl-4,6-O-disulfo-D-glucopyranosid,
Methyl-2-desoxy-2-(3,4-didecyloxybenzoyl)-amino-3-O-tetradecanoyl-4-O-sulfo-D-glucopyranosid,
2-Desoxy-2-tetradecanoylamino-3-O-(3,4-didecyloxybenzoyl)-4-O-sulfo-D-glucopyranose,
2-Desoxy-2-[3-(3,4-dioctyloxy)-phenylpropionyl]-amino-3-O-tetradecanoyl-4-O-sulfo-D-glucopyranose,
Methyl-2-desoxy-2-[3-(3,4-dioctyloxyphenyl)-propanoyl]-amino-3-O-[3-(3,4-dioctyloxyphenyl)-propanoyl]-4,6-disulfo-α-D-glucopyranosid,
Methyl-2-desoxy-2-[3-(3,4-dioctyloxyphenyl)-propanoyl]-amino-3-O-tetradecanoyl-4,6-O-disulfo-α-D-glucopyranosid und
Methyl-2-desoxy-2-tetradecanoylamino-3-O-[3-(3,4-dioctyloxyphenyl)-propanoyl]-4,5-O-disulfo-α-D-glucopyranosid.

6. Verfahren zur Herstellung der Verbindung der allgemeinen Formel:

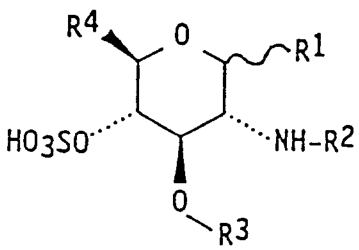

(I)

worin bedeuten:
$R^1$ ein Wasserstoffatom, eine Hydroxygruppe oder eine Alkoxygruppe mit 1 bis 4 Kohlenstoffatom(en),
$R^2$ die Gruppe A, B, D oder E mit
A gleich einer allgemeinen Formel:

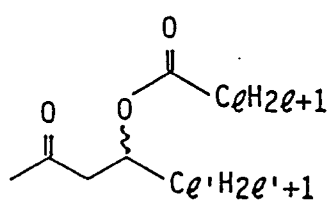

,

B gleich einer allgemeinen Formel:

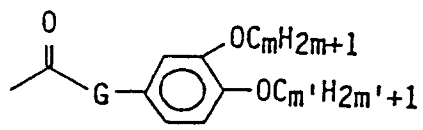
,

D gleich einer allgemeinen Formel:

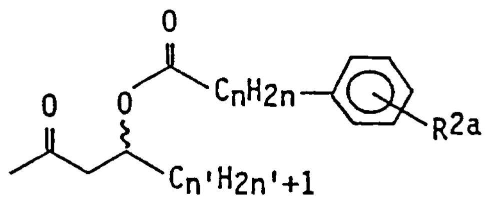

und
E gleich einer allgemeinen Formel:

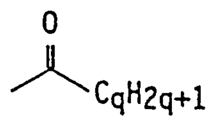
;

wobei in jeder Formel l und q jeweils ganze Zahlen von 11 - 15, m und m' jeweils ganze Zahlen von 6 - 12, n eine ganze Zahl von 6 - 10 und l' und n' jeweils ganze Zahlen von 9 - 13 bedeuten,
G eine Einfachbindung oder eine Alkylengruppe mit 1 - 4 Kohlenstoffatom(en) darstellt und
$R^{2a}$ ein Wasserstoffatom, eine Alkylgruppe oder eine Alkoxygruppe mit 1 bis 7 Kohlenstoffatom(en) oder ein Halogenatom bedeutet,
$R^3$ die Gruppe L, M oder Q mit
L gleich einer allgemeinen Formel:

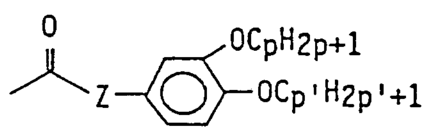
,

M gleich einer allgemeinen Formel:

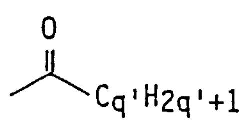

und
Q gleich einer allgemeinen Formel:

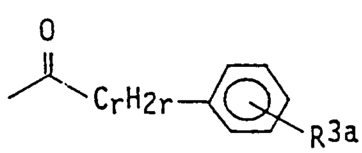
;

wobei in jeder Formel Z eine Einfachbindung oder eine Alkylengruppe mit 1 bis 4 Kohlenstoffatom(en) bedeutet,

p und p' jeweils ganze Zahlen von 6 - 12, q' eine ganze Zahl von 11 - 15 und r eine ganze Zahl von 6 - 10 bedeuten und
$R^{3a}$ für ein Wasserstoffatom, eine Alkylgruppe oder eine Alkoxygruppe mit 1 bis 7 Kohlenstoffatom(en) oder ein Halogenatom steht,
$R^4$ ein Wasserstoffatom, eine Hydroxymethylgruppe oder Sulfoxymethyl ($-CH_2OSO_3H$)-Gruppe, wobei gilt, daß wenn $R^4$ für eine Hydroxymethylgruppe steht,
(i) (A, M), (A,Q), (D,M), (D,Q), (E,M) und (E,Q) als Kombination von ($R^2$, $R^3$) ausgeschlossen sind,
(ii) m und m' in der Formel (B) von $R^2$ für eine ganze Zahl von 8 - 12 stehen und
(iii) p und p' in der Formel (L) von $R^3$ eine ganze Zahl von 8 - 12 bedeuten, und
wenn $R^4$ für eine Sulfoxymethylgruppe steht, (A,M) und (E,M) als Kombination von ($R^2$, $R^3$) ausgeschlossen sind, gekennzeichnet durch Einführen einer Sulfogruppe in die Hydroxygruppe in 4-Stellung einer Verbindung der allgemeinen Formel:

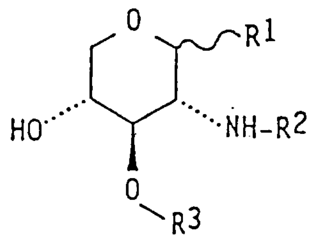

(II)

worin alle Symbole die oben genannte Bedeutung besitzen,
durch Einführen von Sulfogruppen in die Hydroxygruppen in 4- und 6-Stellung einer Verbindung der allgemeinen Formel:

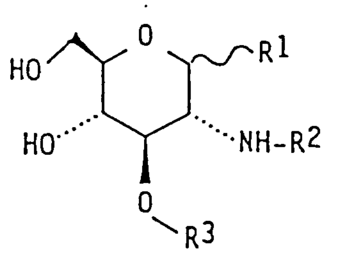

(III)

worin alle Symbole die oben genannte Bedeutung besitzen,
durch Hydrolysieren einer Verbindung der allgemeinen Formel:

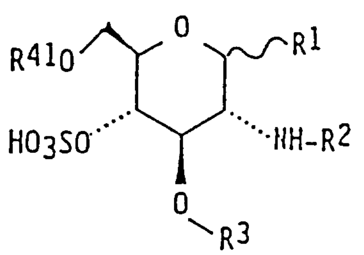

(IV)

worin $R^{41}$ für eine Trialkylsilylgruppe steht und die weiteren Symbole die oben angegebene Bedeutung besitzen,
unter sauren Bedingungen.

**7.** Pharmazeutische Zubereitung zur Verwendung zur Erhöhung der Immunität, gekennzeichnet durch Umfassen einer Verbindung der allgemeinen Formel (I) nach Anspruch 1 als aktiven Bestandteil zusammen mit einem pharmazeutischen Träger und/oder Überzug.

8. Pharmazeutische Zubereitung zur Verwendung bei der Behandlung eines Tumors, gekennzeichnet durch Umfassen einer Verbindung der allgemeinen Formel (I) nach Anspruch 1 als aktiven Bestandteil zusammen mit einem pharmazeutischen Träger und/oder Überzug.

9. Verbindung der allgemeinen Formel (I) nach Anspruch 1 zur Verwendung bei der Herstellung eines Medikaments zur Immunitätserhöhung.

10. Verbindung der allgemeinen Formel (I) nach Anspruch 1 zur Verwendung bei der Herstellung eines Medikaments zur Tumorbehandlung.

**Patentansprüche für folgende Vertragsstaaten : ES, GR**

1. Verfahren zur Herstellung der Verbindung der allgemeinen Formel:

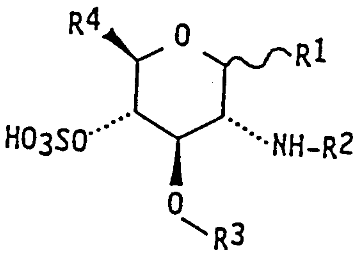

(I)

worin bedeuten:
$R^1$ ein Wasserstoffatom, eine Hydroxygruppe oder eine Alkoxygruppe mit 1 bis 4 Kohlenstoffatom(en),
$R^2$ die Gruppe A, B, D oder E mit
A gleich einer allgemeinen Formel:

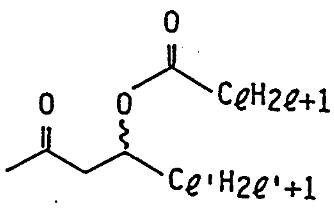

,

B gleich einer allgemeinen Formel:

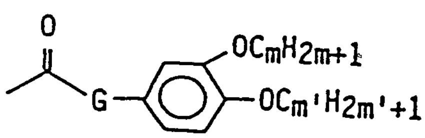

,

D gleich einer allgemeinen Formel:

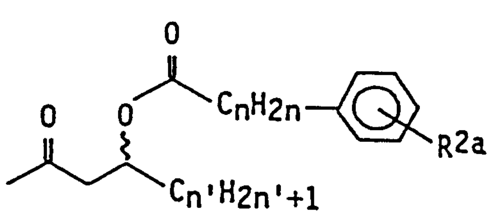

und

E gleich einer allgemeinen Formel:

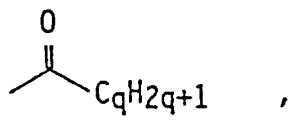

wobei in jeder Formel l und q jeweils ganze Zahlen von 11 - 15, m und m' jeweils ganze Zahlen von 6 - 12, n eine ganze Zahl von 6 - 10 und l' und n' jeweils ganze Zahlen von 9 - 13 bedeuten,
G eine Einfachbindung oder eine Alkylengruppe mit 1 - 4 Kohlenstoffatom(en) darstellt und
$R^{2a}$ ein Wasserstoffatom, eine Alkylgruppe oder eine Alkoxygruppe mit 1 bis 7 Kohlenstoffatom(en) oder ein Halogenatom bedeutet,
$R^3$ die Gruppe L, M oder Q mit
L gleich einer allgemeinen Formel:

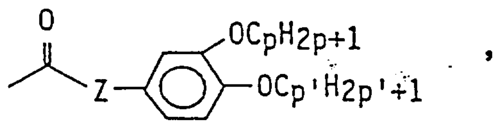

M gleich einer allgemeinen Formel:

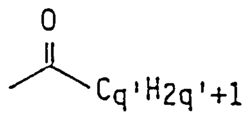

und
Q gleich einer allgemeinen Formel:

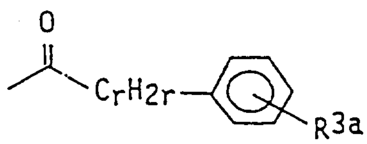

wobei in jeder Formel Z eine Einfachbindung oder eine Alkylengruppe mit 1 bis 4 Kohlenstoffatom(en) bedeutet, p und p' jeweils ganze Zahlen von 6 - 12, q' eine ganze Zahl von 11 - 15 und r eine ganze Zahl von 6 - 10 bedeuten und
$R^{3a}$ für ein Wasserstoffatom, eine Alkylgruppe oder Alkoxygruppe mit 1 bis 7 Kohlenstoffatom(en) oder ein Halogenatom steht,
$R^4$ ein Wasserstoffatom, eine Hydroxymethylgruppe oder Sulfoxymethylgruppe,
wobei gilt, daß wenn $R^4$ für eine Hydroxymethylgruppe steht,

(i) (A, M), (A,Q), (D,M), (D,Q), (E,M) und (E,Q) als Kombination von ($R^2$, $R^3$) ausgeschlossen sind,
(ii) m und m' in der Formel (B) von $R^2$ für eine ganze Zahl von 8 - 12 stehen und
(iii) p und p' in der Formel (L) von $R^3$ eine ganze Zahl von 8 - 12 bedeuten, und

wenn $R^4$ für eine Sulfoxymethylgruppe steht, (A,M) und (E,M) als Kombination von ($R^2$, $R^3$) ausgeschlossen sind, gekennzeichnet durch Einführen einer Sulfogruppe in die Hydroxygruppe in 4-Stellung einer Verbindung der allgemeinen Formel:

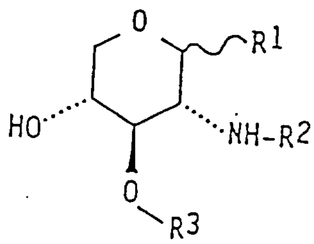

(II)

worin alle Symbole die oben genannte Bedeutung besitzen,
durch Einführen von Sulfogruppen in die Hydroxygruppen in 4- und 6-Stellung einer Verbindung der allgemeinen Formel:

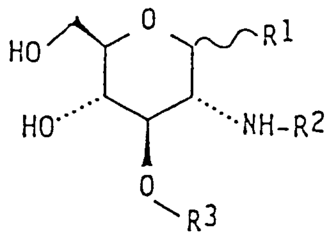

(III)

worin alle Symbole die oben genannte Bedeutung besitzen,
durch Hydrolysieren einer Verbindung der allgemeinen Formel:

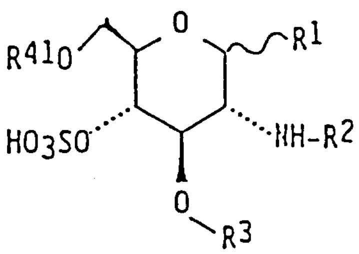

(IV)

worin $R^{41}$ für eine Trialkylsilylgruppe steht und die weiteren Symbole die oben angegebene Bedeutung besitzen,
unter sauren Bedingungen.

2. Verfahren zur Herstellung der Verbindung nach Anspruch 1, wobei $R^4$ für ein Wasserstoffatom steht.

3. Verfahren zur Herstellung der Verbindung nach Anspruch 1 oder Anspruch 2, wobei $R^2$ in der allgemeinen Formel durch B dargestellt ist.

4. Verfahren zur Herstellung der Verbindung nach Anspruch 1 oder Anspruch 3, wobei $R^3$ in der allgemeinen Formel durch L dargestellt ist.

5. Verfahren zur Herstellung der Verbindung nach Ansprüchen 1 bis 4, ausgewählt aus der Gruppe:
2-Desoxy-2-(3-tetradecanoyloxytetradecanoyl)-amino-3-0-tetradecanoyl-4-0-sulfo-1,5-anhydro-D-xylit,
2-Desoxy-2-[3-(9-phenylnonanoyl)-oxytetradecanoyl]-amino-3-0-(9-phenylnonanoyl)-4-0-sulfo-1,5-anhydro-D-xylit,
2-Desoxy-2-(3,4-didecyloxybenzoyl)-amino-3-0-tetradecanoyl-4-0-sulfo-1,5-anhydro-D-xylit,
Methyl-2-desoxy-(3,4-didecyloxybenzoyl)-amino-3-0-tetradecanoyl-4-0-sulfo-5-anhydro-D-glucopyranosid,
2-Desoxy-2-(3,4-didecyloxybenzoyl)-amino-3-0-tetradecanoyl-4-O-sulfo-D-glucopyranose,

2-Desoxy-2-(3,4-didecyloxybenzoyl)-amino-3-O-(3,4-didecyloxybenzoyl)-4-O-sulfo-D-glucopyranose,
Methyl-2-desoxy-2-(3,4-didecyloxybenzoyl)-amino-3-O-(3,4-didecyloxybenzoyl)-4,6-disulfo-α-D-glucopyranosid,
Methyl-2-desoxy-2-(3,4-didecyloxybenzoyl)-amino-3-O-(3,4-didecyloxybenzoyl)-4-O-sulfo-α-D-glucopyranosid,
2-Desoxy-2-(3,4-didecyloxybenzoyl)-amino-3-0-tetradecanoyl-4,6-O-disulfo-D-glucopyranosid,
Methyl-2-desoxy-2-(3,4-didecyloxybenzoyl)-amino-3-O-tetradecanoyl-4-O-sulfo-D-glucopyranosid,
2-Desoxy-2-tetradecanoylamino-3-O-(3,4-didecyloxybenzoyl)-4-O-sulfo-D-glucopyranose,
2-Desoxy-2-[3-(3,4-dioctyloxy)-phenylpropionyl]-amino-3-O-tetradecanoyl-4-O-sulfo-D-glucopyranose,
Methyl-2-desoxy-2-[3-(3,4-dioctyloxyphenyl)-propanoyl]-amino-3-O-[3-(3,4-dioctyloxyphenyl)-propanoyl]-4,6-disulfo-α-D-glucopyranosid,
Methyl-2-desoxy-2-[3-(3,4-dioctyloxyphenyl)-propanoyl]-amino-3-O-tetradecanoyl-4,6-O-disulfo-α-D-glucopyranosid und
Methyl-2-desoxy-2-tetradecanoylamino-3-O-[3-(3,4-dioctyloxyphenyl)-propanoyl]-4,5-O-disulfo-α-D-glucopyranosid.

6. Verfahren zur Herstellung der pharmazeutischen Zubereitung zur Verwendung zur Erhöhung der Immunität, gekennzeichnet durch Umfassen einer Verbindung der allgemeinen Formel (I) nach Anspruch 1 als aktiven Bestandteil zusammen mit einem pharmazeutischen Träger und/oder Überzug.

7. Verfahren zur Herstellung der pharmazeutischen Zubereitung zur Verwendung bei der Tumorbehandlung, gekennzeichnet durch Umfassen einer Verbindung der allgemeinen Formel (I) nach Anspruch 1 als aktiven Bestandteil zusammen mit einem pharmazeutischen Träger und/oder Überzug.

**Revendications**

**Revendications pour les Etats contractants suivants : AT, BE, CH, DE, FR, GB, IT, LI, LU, NL, SE**

1. Un dérivé de glucopyrannose de formule générale :

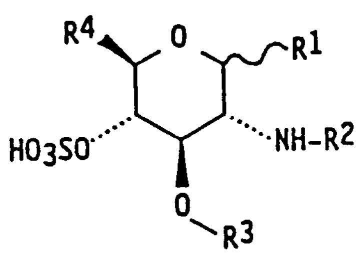

(I)

dans laquelle $R^1$ représente un atome d'hydrogène, un groupe hydroxy ou un groupe alcoxy en $C_1$-$C_4$, $R^2$ représente le groupe représenté par A, B, D ou E, dans lesquels
A répond à la formule générale :

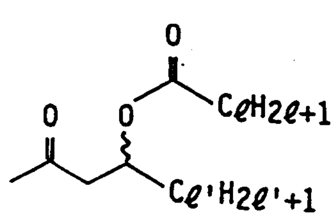

,

B répond à la formule générale :

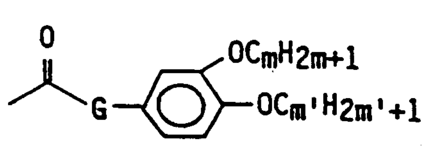

,

D répond à la formule générale :

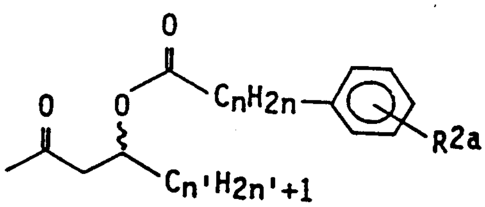

et

E répond à la formule générale :

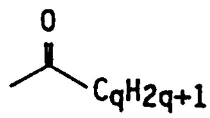

;

dans chaque formule, l et q représentent chacun un entier de 11 à 15, m et m' représentent chacun un entier de 6 à 12, n représente un entier de 6 à 10, l' et n' représentent chacun un entier de 9 à 13,

G représente une liaison simple ou un groupe alkylène en $C_1$-$C_4$,

$R^{2a}$ représente un atome d'hydrogène, un groupe alkyle ou un groupe alcoxy en $C_1$-$C_7$ ou un atome d'halogène,

$R^3$ représente le groupe représenté par L, M ou Q, dans lesquels

L répond à la formule générale :

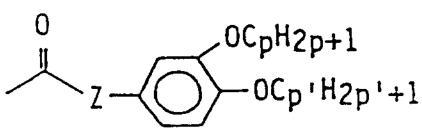

,

M répond à la formule générale :

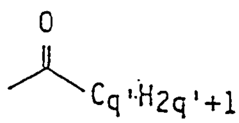

et

Q répond à la formule générale :

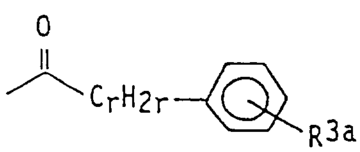

;

dans chaque formule, Z représente une liaison simple ou un groupe alkylène en $C_1$-$C_4$, p et p' représentent chacun un entier de 6 à 12, q' représente un entier de 11 à 15, r représente un entier de 6 à 10, $R^{3a}$ représente un atome d'hydrogène, un groupe alkyle ou un groupe alcoxy en $C_1$-$C_7$ ou un atome d'halogène,

$R^4$ représente un atome d'hydrogène, un groupe hydroxyméthyle ou un groupe sulfoxyméthyle ($-CH_2OSO_3H$),

avec les conditions que, lorsque $R^4$ représente un groupe hydroxyméthyle, (i) (A,M), (A,Q), (D,M),

(D,Q), (E,M) et (E,Q) sont exclues comme combinaisons de ($R^2$, $R^3$), (ii) m et m' dans la formule B de $R^2$ représentent un entier de 8 à 12 et (iii) p et p' dans la formule L de $R^3$ représentent un entier de 8 à 12,

et lorsque $R^4$ représente un groupe sulfoxyméthyle, (A,M) et (E,M) sont exclues comme combinaisons de ($R^2$, $R^3$), ou ses sels d'addition d'acides non toxiques.

2. Un composé selon la revendication 1, dans lequel $R^4$ est un atome d'hydrogène.

3. Un composé selon la revendication 1 ou 2, dans lequel $R^2$ répond à la formule générale représentée par B.

4. Un composé selon la revendication 1 ou 3, dans lequel $R^3$ répond à la formule générale représentée par L.

5. Un composé selon les revendications 1 à 4, qui est choisi parmi les suivants :
2-désoxy-2-(3-tétradécanoyloxytétradécanoyl)amino-3-O-tétradécanoyl-4-O-sulfo-1,5-anhydro-D-xylitol,
2-désoxy-2-[3-(9-phénylnonanoyl)oxytétradécanoyl]amino-3-O-(9-phénylnonanoyl)-4-O-sulfo-1,5-anhydro-D-xylitol,
2-désoxy-2-(3,4-didécyloxybenzoyl)amino-3-O-tétradécanoyl-4-O-sulfo-1,5-anhydro-D-xylitol,
méthyl-2-désoxy-2-(3,4-didécyloxybenzoyl)amino-3-O-tétradécanoyl-4-O-sulfo-5-anhydro-D-glucopyrannoside,
2-désoxy-2-(3,4-didécyloxybenzoyl)amino-3-O-tétradécanoyl-4-O-sulfo-D-glucopyrannose,
2-désoxy-2-(3,4-didécyloxybenzoyl)amino-3-O-(3,4-didécyloxybenzoyl)-4-O-sulfo-D-glucopyrannose,
méthyl-2-désoxy-2-(3,4-didécyloxybenzoyl)amino-3-O-(3,4-didécyloxybenzoyl)-4,6-disulfo-α-D-glucopyrannoside,
méthyl-2-désoxy-2-(3,4-didécyloxybenzoyl)-amino-3-O-(3,4-didécyloxybenzoyl)-4-O-sulfo-α-D-glucopyrannoside,
2-désoxy-2-(3,4-didécyloxybenzoyl)amino-3-O-tétradécanoyl-4,6-O-disulfo-D-glucopyrannoside,
méthyl-2-désoxy-2-(3,4-didécyloxybenzoyl)amino-3-O-tétradécanoyl-4-O-sulfo-D-glucopyrannoside,
2-désoxy-2-tétradécanoylamino-3-O-(3,4-didécyloxybenzoyl)-4-O-sulfo-D-glucopyrannose,
2-désoxy-2-[3-(3,4-dioctyloxyphényl)propionyl]amino-3-O-tétradécanoyl-4-O-sulfo-D-glucopyrannose,
méthyl-2-désoxy-2-[3-(3,4-dioctyloxyphényl)propanoyl]amino-3-O-[3-(3,4-dioctyloxyphényl)propanoyl]-4,6-disulfo-α-D-glucopyrannoside,
méthyl-2-désoxy-2-[3-(3,4-dioctyloxyphényl)propanoyl]amino-3-O-tétradécanoyl-4,6-O-disulfo-α-D-glucopyrannoside et
méthyl-2-désoxy-2-tétradécanoylamino-3-O-[3-(3,4-dioctyloxyphényl)-propanoyl]-4,5-O-disulfo-α-D-glucopyrannoside.

6. Un procédé pour la préparation du composé de formule générale :

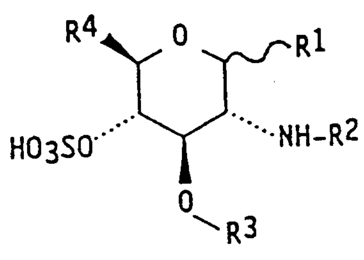

(I)

dans laquelle $R^1$ représente un atome d'hydrogène, un groupe hydroxy ou un groupe alcoxy en $C_1$-$C_4$, $R^2$ représente le groupe représenté par A, B, D ou E, dans lesquels
A répond à la formule générale :

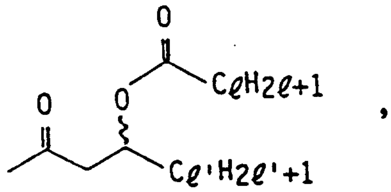

B répond à la formule générale :

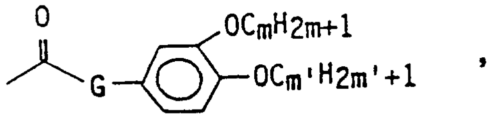

D répond à la formule générale :

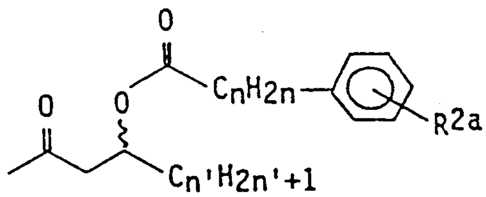

et
E répond à la formule générale :

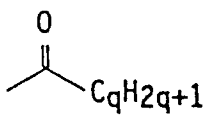

dans chaque formule, l et q représentent chacun un entier de 11 à 15, m et m' représentent chacun un entier de 6 à 12, n représente un entier de 6 à 10, l' et n' représentent chacun un entier de 9 à 13,
G représente une liaison simple ou un groupe alkylène en $C_1$-$C_4$,
$R^{2a}$ représente un atome d'hydrogène, un groupe alkyle ou un groupe alcoxy en $C_1$-$C_7$ ou un atome d'halogène,
$R^3$ représente le groupe représenté par L, M ou Q, dans lesquels
L répond à la formule générale :

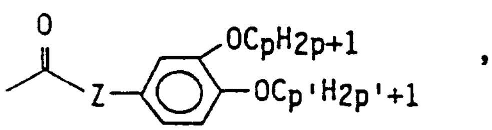

M répond à la formule générale :

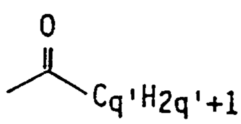

et

Q répond à la formule générale :

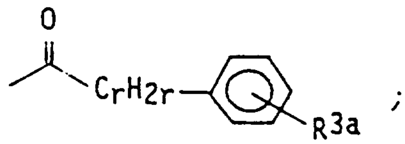

dans chaque formule, Z représente une liaison simple ou un groupe alkylène en $C_1$-$C_4$, p et p' représentent chacun un entier de 6 à 12, q' représente un entier de 11 à 15, r représente un entier de 6 à 10, $R^{3a}$ représente un atome d'hydrogène, un groupe alkyle ou un groupe alcoxy en $C_1$-$C_7$ ou un atome d'halogène,

$R^4$ représente un atome d'hydrogène, un groupe hydroxyméthyle ou un groupe sulfoxyméthyle ($-CH_2OSO_3H$),

avec les conditions que, lorsque $R^4$ représente un groupe hydroxyméthyle, (i) (A,M), (A,Q), (D,M), (D,Q), (E,M) et (E,Q) sont exclues comme combinaisons de ($R^2$, $R^3$), (ii) m et m' dans la formule B de $R^2$ représentent un entier de 8 à 12 et (iii) p et p' dans la formule L de $R^3$ représentent un entier de 8 à 12,

et lorsque $R^4$ représente un groupe sulfoxyméthyle, (A,M) et (E,M) sont exclues comme combinaisons de ($R^2$, $R^3$),

qui est caractérisé

en ce qu'on introduit un groupe sulfo dans le groupe hydroxy en position 4 d'un composé de formule générale :

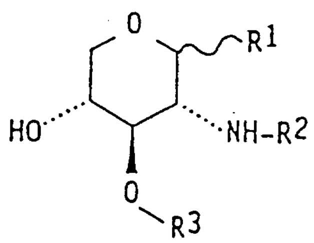

(II)

dans laquelle tous les symboles ont la même signification que précédemment,

en ce qu'on introduit des groupes sulfo dans les groupes hydroxy en positions 4 et 6 d'un composé de formule générale :

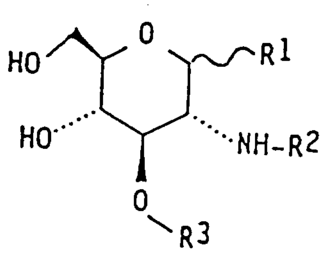

(III)

dans laquelle tous les symboles ont la même signification que précédemment,

en ce qu'on hydrolyse un composé de formule générale :

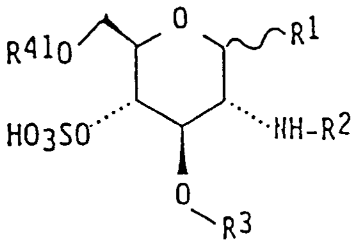

(IV)

dans laquelle $R^{41}$ représente un groupe trialkylsilyle et les autres symboles ont les mêmes significations que précédemment,
en conditions acides.

7. Une composition pharmaceutique à utiliser pour améliorer l'immunité, caractérisée en ce qu'elle comprend comme ingrédient actif un composé de formule générale (I) représentée à la revendication 1 en combinaison avec un support et/ou un revêtement pharmaceutique.

8. Une composition pharmaceutique à utiliser dans le traitement d'une tumeur, caractérisée en ce qu'elle comprend comme ingrédient actif un composé de formule générale (I) représentée à la revendication 1 en combinaison avec un support et/ou un revêtement pharmaceutique.

9. Un composé de formule générale (I) représentée à la revendication 1, à utiliser dans la fabrication d'un médicament pour l'amélioration de l'immunité.

10. Un composé de formule générale (I) représentée à la revendication 1, à utiliser pour la fabrication d'un médicament pour le traitement d'une tumeur.

**Revendications pour les Etats contractants suivants : ES, GR**

1. Un procédé pour la préparation du composé de formule générale :

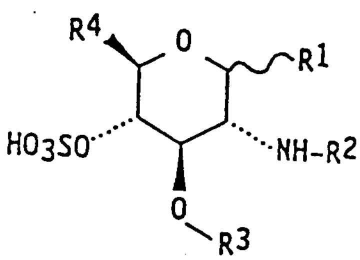

(I)

dans laquelle $R^1$ représente un atome d'hydrogène, un groupe hydroxy ou un groupe alcoxy en $C_1$-$C_4$,
$R^2$ représente le groupe représenté par A, B, D ou E, dans lesquels
A répond à la formule générale :

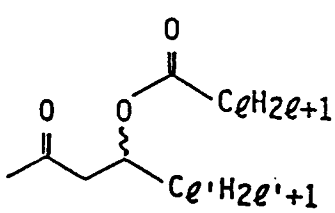

,

B répond à la formule générale :

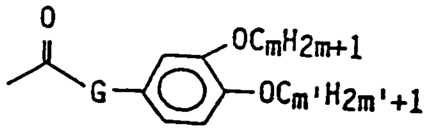,

D répond à la formule générale :

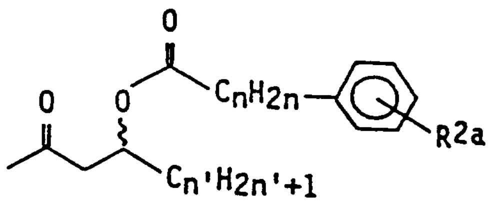

et
E répond à la formule générale :

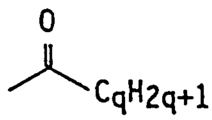

dans chaque formule, l et q représentent chacun un entier de 11 à 15, m et m' représentent chacun un entier de 6 à 12, n représente un entier de 6 à 10, l' et n' représentent chacun un entier de 9 à 13,
G représente une liaison simple ou un groupe alkylène en $C_1$-$C_4$,
$R^{2a}$ représente un atome d'hydrogène, un groupe alkyle ou un groupe alcoxy en $C_1$-$C_7$ ou un atome d'halogène,
$R^3$ représente le groupe représenté par L, M ou Q, dans lesquels
L répond à la formule générale :

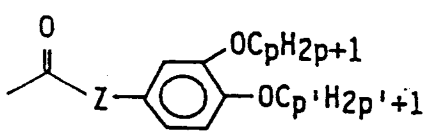,

M répond à la formule générale :

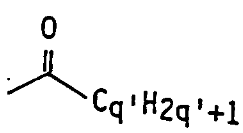

et
Q répond à la formule générale :

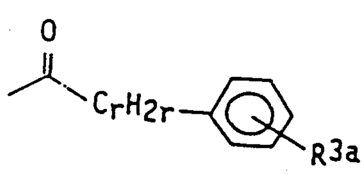

dans chaque formule, Z représente une liaison simple ou un groupe alkylène en $C_1$-$C_4$, p et p'

représentent chacun un entier de 6 à 12, q' représente un entier de 11 à 15, r représente un entier de 6 à 10, $R^{3a}$ représente un atome d'hydrogène, un groupe alkyle ou un groupe alcoxy en $C_1$-$C_7$ ou un atome d'halogène,

$R^4$ représente un atome d'hydrogène, un groupe hydroxyméthyle ou un groupe sulfoxyméthyle (-$CH_2OSO_3H$),

avec les conditions que, lorsque $R^4$ représente un groupe hydroxyméthyle, (i) (A,M), (A,Q), (D,M), (D,Q), (E,M) et (E,Q) sont exclues comme combinaisons de ($R^2$, $R^3$), (ii) m et m' dans la formule B de $R^2$ représentent un entier de 8 à 12 et (iii) p et p' dans la formule L de $R^3$ représentent un entier de 8 à 12,

et lorsque $R^4$ représente un groupe sulfoxyméthyle, (A,M) et (E,M) sont exclues comme combinaisons de ($R^2$, $R^3$),

qui est caractérisé

en ce qu'on introduit un groupe sulfo dans le groupe hydroxy en position 4 d'un composé de formule générale :

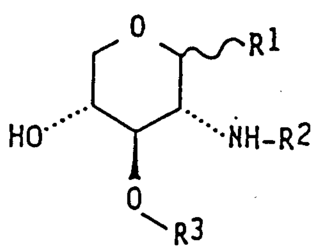

(II)

dans laquelle tous les symboles ont la même signification que précédemment,

en ce qu'on introduit des groupes sulfo dans les groupes hydroxy en positions 4 et 6 d'un composé de formule générale :

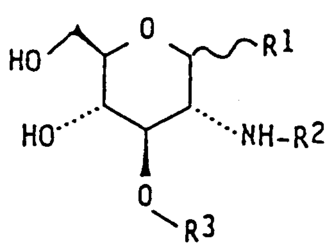

(III)

dans laquelle tous les symboles ont la même signification que précédemment,

en ce qu'on hydrolyse un composé de formule générale :

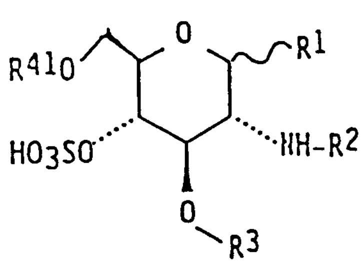

(IV)

dans laquelle $R^{41}$ représente un groupe trialkylsilyle et les autres symboles ont les mêmes significations que précédemment,

en conditions acides.

2. Un procédé pour la préparation du composé selon la revendication 1, dans lequel $R^4$ est un atome d'hydrogène.

3. Un procédé pour la préparation du composé selon la revendication 1 ou 2, dans lequel $R^2$ répond à la formule générale représentée par B.

4. Un procédé selon la revendication 1 ou 3, dans lequel $R^3$ répond à la formule générale représentée par L.

5. Un procédé pour la préparation du composé selon les revendications 1 à 4, qui est choisi parmi les suivants :
2-désoxy-2-(3-tétradécanoyloxytétradécanoyl)amino-3-O-tétradécanoyl-4-O-sulfo-1,5-anhydro-D-xylitol,
2-désoxy-2-[3-(9-phénylnonanoyl)oxytétradécanoyl]amino-3-O-(9-phénylnonanoyl)-4-O-sulfo-1,5-anhydro-D-xylitol,
2-désoxy-2-(3,4-didécyloxybenzoyl)amino-3-O-tétradécanoyl-4-O-sulfo-1,5-anhydro-D-xylitol,
méthyl-2-désoxy-2-(3,4-didécyloxybenzoyl)amino-3-O-tétradécanoyl-4-O-sulfo-5-anhydro-D-glucopyrannoside,
2-désoxy-2-(3,4-didécyloxybenzoyl)amino-3-O-tétradécanoyl-4-O-sulfo-D-glucopyrannose,
2-désoxy-2-(3,4-didécyloxybenzoyl)amino-3-O-(3,4-didécyloxybenzoyl)-4-O-sulfo-D-glucopyrannose,
méthyl-2-désoxy-2-(3,4-didécyloxybenzoyl)amino-3-O-(3,4-didécyloxybenzoyl)-4,6-disulfo-α-D-glucopyrannoside,
méthyl-2-désoxy-2-(3,4-didécyloxybenzoyl)-amino-3-O-(3,4-didécyloxybenzoyl)-4-O-sulfo-α-D-glucopyrannoside,
2-désoxy-2-(3,4-didécyloxybenzoyl)amino-3-O-tétradécanoyl-4,6-O-disulfo-D-glucopyrannoside,
méthyl-2-désoxy-2-(3,4-didécyloxybenzoyl)amino-3-O-tétradécanoyl-4-O-sulfo-D-glucopyrannoside,
2-désoxy-2-tétradécanoylamino-3-O-(3,4-didécyloxybenzoyl)-4-O-sulfo-D-glucopyrannose,
2-désoxy-2-[3-(3,4-dioctyloxyphényl)propionyl]amino-3-O-tétradécanoyl-4-O-sulfo-D-glucopyrannose,
méthyl-2-désoxy-2-[3-(3,4-dioctyloxyphényl)propanoyl]amino-3-O-[3-(3,4-dioctyloxyphényl)propanoyl]-4,6-disulfo-α-D-glucopyrannoside,
méthyl-2-désoxy-2-[3-(3,4-dioctyloxyphényl)propanoyl]amino-3-O-tétradécanoyl-4,6-O-disulfo-α-D-glucopyrannoside et
méthyl-2-désoxy-2-tétradécanoylamino-3-O-[3-(3,4-dioctyloxyphényl)propanoyl]-4,5-O-disulfo-α-D-glucopyrannoside.

6. Un procédé pour la préparation d'une composition pharmaceutique à utiliser dans l'amélioration de l'immunité, caractérisé en ce qu'elle comprend comme ingrédient actif un composé de formule générale (I) représentée à la revendication 1 en combinaison avec un support et/ou un revêtement pharmaceutique.

7. Un procédé pour la préparation d'une composition pharmaceutique à utiliser dans le traitement d'une tumeur, caractérisé en ce qu'elle comprend comme ingrédient actif un composé de formule générale (I) représentée à la revendication 1 en combinaison avec un support et/ou un revêtement pharmaceutique.